# EUROPEAN PATENT APPLICATION

(11) **EP 3 501 508 A1**
(43) Date of publication of application: **26.06.2019**
(21) Application number: 17382901.1
(22) Date of filing: 22.12.2017
(51) Int. Cl.: A61K 9/51, A61K 47/69

(54) **THERAPEUTIC NANOPARTICLES**

(71) Applicant: Fundación Centro Nacional De Investigaciones Oncológicas Carlos III, 28029 Madrid (ES); Universitat Politècnica de València, 46022 Valencia (ES)
(72) Inventor: SERRANO MARUGÁN, Manuel, 28760 Tres Cantos, Madrid (ES); MUÑOZ ESPÍN, Daniel, Cambridge, Cambridgeshire CB1 8YU (GB); ROVIRA DEL OLMO, Miguel, 28033 Madrid (ES); BERNARDOS BAU, Andrea, 46111 Rocafort, Valencia (ES); GALIANA GUILLEM, Irene, 46540 El Puig, Valencia (ES); LOZANO TORRES, Beatriz, 46520 Puerto de Sagunto, Valencia (ES); MARTINEZ MAÑEZ, Ramón, 46133 Meliana, Valencia (ES); SANCENÓN GALARZA, Félix, 46940 Manises, Valencia (ES)
(74) Representative: Elzaburu S.L.P.

(57) **Abstract**

The invention relates to a nanoparticle conjugate arranged, in use, to encapsulate and deliver a payload molecule to a damaged and/or senescent cell. Therapeutic uses thereof and cosmetic and imaging applications are also provided. The nanoparticle preferably presents a homogeneous coating of 6-mer galacto-oligosaccharides at each cavity on its surface and can be for use in the treatment, amelioration, or prevention of a disease or condition characterised by the presence of damaged and/or senescent cells.

SEQUENCE LISTING

<110>  Fundación Centro Nacional de Investigaciones Oncológicas
       Carlos III
       Universitat Politècnica de València

<120>  Therapeutic Nanoparticles

<130>  EP-1701

<160>  10

<170>  PatentIn version 3.5

<210>  1
<211>  21
<212>  DNA
<213>  Artificial sequence

<220>
<223>  Primer Actb Forward

<400>  1
ggcaccacac cttctacaat g                                                21

<210>  2
<211>  20
<212>  DNA
<213>  Artificial sequence

<220>
<223>  Primer Actb Reverse

<400>  2
gtggtggtga agctgtagcc                                                  20

<210>  3
<211>  20
<212>  DNA
<213>  Artificial sequence

<220>
<223>  Primer Gapdh Forward

<400>  3
ttcaccacca tggagaaggc                                                  20

<210>  4
<211>  18
<212>  DNA
<213>  Artificial sequence

<220>
<223>  Primer Gapdh Reverse

<400>  4
   cccttttggc tccaccct                                                   18

<210>  5

<211>  20
<212>  DNA
<213>  Artificial sequence

<220>
<223>  Primer Il6 Forward

<400>  5
gttctctggg aaatcgtgga                                                    20


<210>  6
<211>  22
<212>  DNA
<213>  Artificial sequence

<220>
<223>  Primer Il6 Reverse

<400>  6
ggtactccag aagaccagag ga                                                 22


<210>  7
<211>  20
<212>  DNA
<213>  Artificial sequence

<220>
<223>  Primer Myh7 Forward

<400>  7
tgcagcagtt cttcaaccac                                                    20


<210>  8
<211>  20
<212>  DNA
<213>  Artificial sequence

<220>
<223>  Primer Myh7 Reverse

<400>  8
tcgaggcttc tggaagttgt                                                    20


<210>  9
<211>  20
<212>  DNA
<213>  Artificial sequence

<220>
<223>  Primer Nppa Forward

<400>  9
atctgccctc ttgaaaagca                                                    20


<210>  10
<211>  20
<212>  DNA
<213>  Artificial sequence

<220>
<223>  Primer Nppa Reverse

<400>  10
acacaccaca agggcttagg                                                    20

## Description

The invention relates to a nanoparticle conjugate arranged, in use, to encapsulate and deliver a payload molecule to a damaged and/or senescent cell. Pharmaceutical compositions, medicaments and therapeutic uses thereof are also provided. The invention also extends to a variety of cosmetic and imaging applications.

Targeting cellular senescence remains a challenge in medicine. Besides being relevant for ageing, cellular senescence is associated with a variety of diseases, including cancer and fibrotic disorders.

Severe or un-repairable cellular damage often triggers a stereotypic cellular response known as senescence, controlled by relatively well-understood pathways that stably block cell proliferation and are conserved across vertebrates (Muñoz-Espín and Serrano, 2014).

Evidences suggest that the biological purpose of this response is the orchestration of tissue repair, which occurs as a consequence of the intense secretion of a complex and heterogeneous mixture of extracellular factors known as Senescence Associated Secretory Phenotype (SASP). Specifically, senescence-initiated tissue repair involves the recruitment of inflammatory cells, the activation of proliferation in non-damaged surrounding cells, and eventually the dismissal of senescent cells by phagocytic and immune cells (Lujambio, 2016). Even during embryonic development, embryos use senescence in a programmed manner in response to developmental cues to initiate specific organogenic remodelling processes (Muñoz-Espín *et al.,* 2013; Storer *et al.,* 2013). However, this dynamic and transient process may go awry and senescent cells may reside in tissues for extended periods of time, resulting in the progressive accumulation of senescent cells and the associated processes of inflammation and fibrosis, as happens in many human diseases and also in aged tissues (Muñoz-Espín and Serrano, 2014). Indeed, recent evidence in mice indicates that the accumulation of senescent cells is an active pathological component of multiple diseases and aging. Consequently, genetic ablation of senescent cells has been demonstrated to ameliorate some aging-associated diseases, revert long-term degenerative processes associated with chemotherapy, and extend longevity (Baker *et al.,* 2016; Childs *et al.,* 2016; Demaria *et al.,* 2016).

Several drugs with senolytic (senescence destroying) activity have been identified and investigated with respect to their efficacy in extending lifespan and alleviating age-associated disorders in mouse models.

Examples of these senolytic drugs include dasatinib, a tyrosine kinase inhibitor developed by Bristol-Myers Squibb for the treatment of chronic leukemia, and quercetin, a flavonol that has phosphatidyl inositol 3 kinase (PI₃K) inhibitory activity. Used in combination, these drugs reduced the number of damaged and/or senescent cells in chronologically aged mice, in progeroid mice, and in irradiation-exposed mice, thereby resulting in improved cardiac and vascular function, delayed aged-related symptoms and extended lifespan (Zhu *et al.,* 2015; Roos *et al.,* 2016). In addition, ABT-737, an inhibitor of the anti-apoptotic proteins BCL-W and BCL-XL, both of which are up-regulated by senescent cells, has been shown to selectively eliminate senescent cells and, as a result, rejuvenate hematopoietic stem cells in irradiated or naturally-aged mice (Yosef *et al.,* 2016). Another inhibitor of the Bcl-2 family, navitoclax (ABT-263), has also been shown to efficiently eliminate senescent cells *in vitro* and in *vivo* (Chang *et al.,* 2016; Zhu *et al.,* 2016). Pharmacological elimination of senescent cells has also been shown to alleviate atherosclerosis symptoms (Childs *et al.,* 2016; Roos *et al.,* 2016) and disc degeneration (Feng *et al.,* 2016) in mice.

Although the above-mentioned therapies have been demonstrated to be effective in animal models with respect to damaged and/or senescent cells, they have never been tested to evaluate whether they have the same effectiveness in humans. Rather, they are currently only being evaluated in human clinical trials as anti-cancer therapies. The reason for this is that all of the above treatments also induce an apoptotic response in healthy cells, as well as tumor and/or damaged and/or senescent cells. This apoptotic response in healthy cells is commonly associated with most anti-cancer therapies and is responsible for the often debilitating side effects routinely associated with standard chemotherapy. Consequently, the overall benefit gained in using these therapies to treat chronic diseases, or as preventative interventions, should be carefully assessed.

There is, therefore, a need in the art for a method capable of eliminating damaged and/or senescent cells, but without the cytotoxic side-effects associated with the therapies referred to above.

Senescent cells are characterized by high levels of lysosomal-associated *β*-galactosidase activity (SA-*β*gal) and lysosomal-associated *α*-fucosidase activity (SA-*α*fuc) (Dimri *et al.,* 1995; Knas *et al.,* 2012; Muñoz-Espín and Serrano, 2014). Although SA-*β*gal is the first and most widely used marker of senescence, the biological basis of SA-*β*gal still remain largely speculative. A number of reports have described abnormal endosomal traffic in senescent cells, including aberrant autophagy and accumulation of lysosomes (Cho *et al.,* 2011; Narita *et al.,* 2011; Ivanov *et al.,* 2013; Udono *et al.,* 2015; Tai *et al.,* 2017), which may be related to the high secretory activity of senescent cells. Indeed, recent data has shown that inhibition of SASP also results in inhibition of SA-*β*gal (Herranz *et al.,* 2015; Laberge *et al.,* 2015).

The present inventors have surprisingly found that it is possible to eliminate damaged and/or senescent cells using therapies of the type referred to above, but without incurring the cytotoxic side-effects routinely associated with these therapies. They have achieved this by using SA-*β*gal and SA-*α*fuc as a marker of vulnerability of senescent cells that can be exploited to deliver cargos, payloads, tracers/markers or drugs preferentially to damaged and/or senescent cells. The inventors' approach consists of encapsulating diagnostic and/or therapeutic agents within a nanoparticle and ensuring that the agent is only released upon contact with a damaged and/or senescent cell. They have shown that this delivery strategy is effective in *vivo* and has the unexpected benefit of reducing known side-effects.

According to a first aspect of the invention, there is provided a nanoparticle arranged, in use, to encapsulate and deliver a payload molecule to a damaged and/or senescent cell, the nanoparticle comprising at least one external cavity in which a payload molecule is contained, the at least one cavity being gated by one or more oligosaccharides, wherein each cavity on said nanoparticle is gated by the same one or more oligosaccharides. In the context of the present invention an "external cavity" is a cavity or reservoir which is externally accessible prior to the gate being applied.

Here, the inventors report pre-clinical therapeutic activity of nanoparticles that preferentially release their payloads upon contact with damaged and/or senescent cells. In particular, both the nanoparticle and payload are internalized by said cell, whereupon the nanoparticles release their payload. Systemic administration of nanoparticles carrying a fluorophore has been found by the inventors to result in the fluorescent labelling of senescent lesions in mice. Moreover, the nanoparticles have been shown by the inventors to target senescent cells in bleomycin-induced pulmonary fibrosis, reduce collagen deposition, and ameliorate pulmonary function. Accordingly, the present invention opens up new diagnostic and therapeutic methods that can be used to diagnose and treat the multiple disorders known to be associated with damaged and/or senescent cells.

The term "nanoparticle" can mean a particle having one or more dimensions at the nanometer scale, i.e. particles having one or more dimensions of between about 0.1 nm and about 1000 nm. The term "nanoparticle" can encompass both ultrafine particles (from about 1 nm to about 100 nm) and fine particles (from about 100 nm to about 500 nm). The average diameter of the nanoparticle may, therefore, be between 1 nm and about 1000 nm, between mm and 750 nm, between 1 nm and 500 nm, between 1 nm and 250 nm or between 1 nm and 100 nm. Preferably, the average diameter of the nanoparticle is between 1 nm and about 500 nm, between 10 nm and 250 nm, between 25 nm and 50 nm, between 25 nm and 100 nm.

Preferably the nanoparticles of the present invention are mesoporous silica nanoparticles.

However, nanoparticles made from many other materials can be used. External cavity means a cavity or reservoir that is in communication with or connected with the outside of the nanoparticle, such that the cavity is externally accessible or can be seen from the outside of, and on the surface of, the nanoparticle using appropriate magnification means.

Preferably, the cavity is a pore with a diameter of between 1 and 10 nm.

A single nanoparticle may comprise 1, 10, 20, 30, 40, 50, 60, 70, 80, 90, 100, 250, 500, 750, 1000, 1200, 1300, 1400, 1500, 1600, 1700, 2000, 2200, 2400 or more external cavity/cavities. Preferably, the nanoparticle comprises around 1500 external cavities.

The cavity/cavities of the nanoparticle comprises/comprise a payload molecule as its/their cargo. The payload molecule may be a bioactive compound, which preferably remains in an active state while it is encapsulated within the cavity. The cavity may be substantially aqueous. For example, the payload molecule may be selected from a group of molecules consisting of an imaging agent, dye, electrochemical mediator, protein, peptide, chemical compound (such as a drug, which may be cytotoxic compound and/or senolytic compound), genetic material (such as an oligonucleotide, DNA, RNA, mRNA, RNAi), small molecule, antibody, enzyme and other bioactive molecule. The cargo may comprise more than one of the aforementioned molecules.

The term "cytotoxic compound" means a compound which is toxic for cells. In contact with cytotoxic compounds, the cells may suffer necrosis, apoptosis or may cease to grow and divide. Examples of cytotoxic compounds valid in the context of the invention would be wherein the core of the cytotoxic is a mitotic inhibitor, such as vinca alkaloids, including vincristine, vinblastine, vinorelbine, vindesine, vinflunine; podophyllotoxin; taxanes, including docetaxel, larotaxel, ortataxel, paclitaxel, and tesetaxel; epothilones, such as ixabepilone; topoisomerase I inhibitors, such as topotecan, irinotecan, camptothecin, rubitecan, and belotecan; topoisomerase type II inhibitors, including amsacrine, etoposide, etoposide phosphate, and teniposide; anthracyclines, such as aclarubicin, daunorubicin, doxorubicin, epirubicin, idarubicin, amrubicin, pirarubicin, valrubicin, and zorubicin; anthracenediones, such as mitoxantrone and pixantrone; antimetabolites, including dihydrofolate reductase inhibitors, such as aminopterin, methotrexate, pemetrexed; thymidylate synthase inhibitors, such as raltitrexed and pemetrexed; adenosine deaminase inhibitors, including pentostatin, halogenated; or ribonucleotide reductase inhibitors, such as cladribine, clofarabine, and fludarabine; thiopurines, including thioguanine and mercaptopurine; thymidylate synthase inhibitors, including fluorouracil, capecitabine, tegafur, carmofur, and floxuridine; DNA polymerase inhibitors, such as cytarabine; ribonucleotide reductase inhibitors, such as gemcitabine; hypomethylating agents, including azacitidine, and decitabine; and ribonucleotide reductase inhibitors, such as hydroxyurea; cell-cycle nonspecific antineoplastic agents, including alkylating agents such as nitrogen mustards, including mechlorethamine, cyclophosphamide, ifosfamide, trofosfamide, chlorambucil, melphalan, prednimustine, bendamustine, uramustine, estramustine; nitrosoureas, including carmustine, lomustine, semustine, fotemustine; nimustine, ranimustine, and streptozocin; alkyl sulfonates, including busulfan, mannosulfan, and treosulfan; aziridines, including carboquone, thioTEPA, triaziquone, and triethylenemelamine; alkylating-like agents, including platinum agents such as cisplatin, carboplatin, oxaliplatin, nedaplatin, triplatin tetranitrate, satraplatin, hydrazines, such as procarbazine, triazenes, such as dacarbazine, temozolomide, altretamine, and mitobronitol, and streptomycins, such as actinomycin, bleomycin, daunomycin, mitomycin, and plicamycin; photosensitizers, including aminolevulinic acid, methyl aminolevulinate, efaproxiral, and porphyrin derivatives, such as porfiiner sodium, talaporfin, temoporfin, and verteporfin; kinase inhibitors such as sorafenib, bosutinib, neratinib, lapatinib, nilotinib, erlotinib, gefitinib, sunitinib, and imatinib; enzyme inhibitors, including farnesyltransferase inhibitors such as tipifarnib, cyclin-dependent kinase inhibitors, such as alvocidib and seliciclib, proteasome inhibitors, such as bortezomib, phosphodiesterase inhibitors, such as anagrelide, IMP dehydrogenase inhibitors, such as tiazofurine, lipoxygenase inhibitors, such as masoprocol, and PARP inhibitors, such as olaparib; receptor antagonists, such as endothelin receptor antagonists including atrasentan, retinoid X receptor antagonists, such as bexarotene, and testolactone; estrogen receptor antagonists, such as tamoxifen; hedgehog inhibitors such as vismodegib; histone deactylase inhibitors such as vorinostat, romidepsin; B-RAF inhibitors such as vemurafenib; estrogen receptor modulators such as raloxifene; and other chemotherapeutics, including mitatane, amsacrine, trabectedin, retinoids such as alitretinoin and tretinoin, arsenic trioxide, asparagine depleters such as asparaginase or pegaspargase, celecoxib, demecolcine, elesclomol, elsamitrucin, etoglucid, and lonidamine.

The term "senolytic compound" refers to a small molecule that can selectively induce death of senescent cells. Senolytic compounds comprise at least one of the listed compounds: quercetin, dasatinib, navitoclax, obatoclax, veliparib, ABT-737, ABT-199 and all the anti-cancer drugs acting as a PARP, Bcl-2, Bcl-XL and Bcl-w inhibitors.

The fact that, in accordance with the present invention, the at least one external cavity is "gated" by at least two saccharides means that access to the cavity is strictly controlled by the saccharides, such that a payload molecule contained within the cavity is not made available until the saccharides are removed, hydrolyzed or degraded in some way. "Gated" is a term of art routinely used in biology and is often used in relation to, for example, gated channels, where access through a membrane is only possible in response to the binding of a chemical messenger.

The nanoparticles of the present invention can selectively deliver their payload to damaged and/or senescent cells, to alter their activity, and/or cause their removal, and may also detect and/or mark damaged and/or senescent cells. Damaged and/or senescent cells present in a specific manner an overexpression of endogenous liposomal β-galactosidase and/or α-fucosidase. This β-galactosidase and/or α-fucosidase is capable of hydrolyzing the saccharides, galactose and fucose respectively, which are present on the nanoparticle, thus releasing the payload.

A homogeneous distribution is one that is uniform in composition or character. The concept is the same with respect to every level of complexity, from atoms to populations of animals or people, or galaxies. Hence, an element may be homogeneous on a larger scale, compared to being heterogeneous on a smaller scale. Accordingly, when applied to the present invention, this means that the more than one oligosaccharides may be homogeneous on a larger scale, compared with being heterogeneous on a smaller scale. However, it is important to note that they may also be homogeneous on a smaller scale as well.

The homogeneous distribution of oligosaccharide(s) present on the surface of the nanoparticle of the present invention advantageously ensures a more controlled release of the payload/cargo from the cavity.

In one embodiment, the oligosaccharide(s) comprise at least one unit of monosaccharide each, with the general formula CₙH₂ₙOₙ.

In another embodiment, the oligosaccharide(s) comprises between 1 and 20 units, or between 1 and 50 units, or between 1 and 100 units, or between 1 and 15 units, or between 5 and 10 units, of monosaccharide each. Preferably, the oligosaccharide comprises 6 monosaccharides. Preferably, the monosaccharide is galactose and/or fucose. Preferably, at least 30%, 40%, 50%, 60%, 70%, 80%, 90%, 95% or 100% of the unit/s of monosaccharide may be galactose and/or fucose.

At least 30% of the monosaccharides being galactose and/or fucose means that if there are 2 monosaccharides, 1 or 2 of them will be galactose and/or fucose; if there are 3 monosaccharides, 2 or 3 of them will be galactose and/or fucose; if there are 4 monosaccharides, 2, 3 or 4 of them will be galactose and/or fucose; if there are 5 monosaccharides, 3, 4 or 5 will be galactose and/or fucose and if there are 6 monosaccharides, 3, 4, 5 or 6 of them will be galactose and/or fucose, and so on. Preferably all the monosaccharides will be galactose and/or fucose.

In one embodiment, the nanoparticle comprises galactose and/or fucose oligosaccharides.

In an embodiment the nanoparticle comprises between 1 and 10000, or between 2000 and 8000, or between 2000 and 6000, or between 3000 and 5000, or between 3500 and 5500, or between 4000 and 5000, preferably 4500 oligosaccharides.

The monosaccharides/oligosaccharide(s) can be the same or different with respect to length and composition, as long as the nanoparticle comprises a homogeneous distribution of them on its surface.

Preferably, the at least one cavity is gated by at least 1, 2, 3, 4, 6, 8, 10 or more saccharides.

Preferably, the cavity is gated by between 1 and 4 saccharides, preferably 3 saccharides.

In an embodiment, the nanoparticle surface is substantially coated with oligosaccharides.

Preferably, at least 55%, 65%, 75%, 85%, 95% or 100% of the nanoparticle surface is coated with oligosaccharides.

Most preferably, a homogeneous coating of 6-mer galacto-oligosaccharides is present on the surface of the nanoparticles of the present invention at the location of the cavity.

Preferably, the oligosaccharides form a self-assembled monolayer (SAM) on the surface of the nanoparticle.

According to a second aspect, there is provided a nanoparticle according to the first aspect of the present invention, wherein the nanoparticle is produced by:-
(i) creating a nanoparticle with at least one cavity;
(ii) loading the at least one cavity with a payload; and
(iii) coating the loaded nanoparticle with oligosaccharide(s), so that the cavity is gated with one or more oligosaccharide.

Thus, according to a third aspect, the invention provides a method of preparing a nanoparticle according to the first aspect, the method comprising:-
(i) creating a nanoparticle with at least one cavity;
(ii) loading the at least one cavity with a payload; and
(iii) coating the loaded nanoparticle with oligosaccharide, so that the cavity is gated with one or more oligosaccharide.

According to a fourth aspect of the invention, there is provided a cell comprising a nanoparticle arranged, in use, to carry a payload molecule to a damaged and/or senescent cell, the nanoparticle comprising at least one external cavity or reservoir, in which a payload molecule is contained, wherein the nanoparticle is one according to the invention described herein.

The cell may be bacterial or animal or a yeast cell. The cell may be mammalian, for example, human. Preferably, the cell is derived from the same species as a subject to which the cell is to be ultimately administered, and may be autologous. Any cell which will be of therapeutic benefit to a subject is contemplated. For example, the cell may be a neuroblastoma cell, a dendritic cell, a macrophage cell or a fibroblast cell. In a particularly preferred embodiment, the cell may be a stem cell.

In one embodiment, the nanoparticle may be introduced into the cell by adding it unchanged and unmodified directly to cell culture medium. As a result of the nanoparticle being present in the cell media, internalization by the cell of the nanoparticle is observed. Thus, the nanoparticle may be internalized into the cell.

However, in another embodiment, the nanoparticle may not be internalized in the cell, and the nanoparticle may, instead, adhere to the cell's surface.

In one embodiment, the nanoparticle may comprise a payload molecule (e.g. a drug molecule). Thus, the nanoparticle may be as described in relation to the first aspect. The payload molecule may be loaded inside the nanoparticle's at least one external cavity and be transported together with the cell to target damaged and/or senescent cells. Release of the nanoparticle from the cell may be initiated externally, which would result in release of the nanoparticle into the surrounding target site. Cell debris, being from the same organism, would then be digested.

However, in another embodiment, the nanoparticle may not comprise a payload molecule, and it is the cell itself which provides the therapeutic effect. Rather, the nanoparticle directs the cell to the damaged and/or senescent cells.

In a fifth aspect of the invention, there is provided a nanoparticle according to the first aspect, or a cell according to the fourth aspect, for use in eliminating damaged and/or senescent cells.

In a sixth aspect of the invention, there is provided a nanoparticle according to the first aspect, or a cell according to the fourth aspect, for use in the treatment, amelioration, or prevention of a disease or condition characterized by the presence of damaged and/or senescent cells.

In a seventh aspect of the invention, there is provided a method of eliminating damaged and/or senescent cells, the method comprising administering to a subject in need of such a treatment, a therapeutically effective amount of a nanoparticle according to the first aspect or a cell according to the fourth aspect.

In an eighth aspect of the present invention, there is provided a method of treating, ameliorating or preventing a disease or condition characterized by the presence of damaged and/or senescent cells, the method comprising administering to a subject in need of such treatment, a therapeutically effective amount of a nanoparticle according to the first aspect or a cell according to the fourth aspect.

The present invention can, therefore, be used to treat, ameliorate or preventing a disease or condition that results from overexpression of *β*-galactosidase and/or *α*-fucosidase, and which preferably takes place in senescent cells. Preferably, the disease or condition is Wiedemann-Rautenstrauch syndrome of neonatal progeria, the Werner syndrome of adult progeria, Hutchinson-Gilford syndrome, Rothmund Thompson syndrome, Mulvill-Smith syndrome, Cockayne syndrome, Dyskeratosis Congenita, pulmonary fibrosis (idiopathic or not), liver fibrosis, renal fibrosis, oral mucous fibrosis, cardiac fibrosis, pancreatic fibrosis, aplastic anaemia, emphysema, type 2 diabetes, degeneration of cartilage, obesity, Alzheimer's disease, Parkinson's disease, sarcopenia, osteoarthritis, intervertebral disc degeneration, macular degeneration, glaucoma, cataracts, pulmonary hypertension, chronic obstructive pulmonary disease, renal transplantation, oral mucositis, or intestinal bowel disease.

The nanoparticles described herein can also be used for the removal of senescent cells following treatment with anti-retroviral drugs, which can cause subcutaneous lipolysis, metabolic dysfunction, and accelerated ageing. Examples of these antiretroviral drugs may include abacavir, azacytidine, azidothymidine (AZT), dideoxycytidine, efavirenz, invirase, ixabepilone, lamivudine (3TC), lopinavir, nevirapine, ritonavir, stavudine, tenofovir, tipranavir, or thiotepa.

The present invention also relates to the use of the nanoparticles as described above, for the removal of senescent cells following treatment with corticoids including cortisol, prednisone, prednisolone, dexamethasone, triamcinolone, beclometasone, fludrocortisone, deoxycorticosterone, and aldosterone. Chronic treatment with these corticoids can lead to cellular senescence and tissue degeneration, for example in tendons following dexamethasone administration, metabolic dysfunction such as obesity and insulin resistance.

The present invention also relates to the use of the nanoparticles as described above, for the removal of senescent cells following long term use of PPAR gamma agonists, including thiazolidinediones such as rosiglitazone, pioglitazone, and troglitazone. The removal of senescent cells will prevent the induction of the SASP, which may be associated with increased adverse cardiac events, and increased risk of cancer.

The present invention also relates to the use of the nanoparticles as described above for the treatment of cellular hypertrophy, including cardiac hypertrophy. Senescent cells retain growth factor signaling, but being unable to undergo division, continue to enlarge and undergo hypertrophy. Senescent cells frequently experience constitutive mammalian target of rapamycin (mTOR) complex activation. Removal of senescent cells with the present therapy will treat diseases caused by cellular senescence, for example, cardiac hypertrophy.

The present invention relates to the use of the nanoparticles as described above, for the treatment of chronic inflammation, caused by cytokines released as part of the SASP from senescent cells.

The nanoparticles can also be used to reduce the side effects of, and accelerated ageing caused by, exposure to radiation, which causes DNA damage and cellular senescence, with subsequent release of SASP cytokines. Senescence may be caused by various forms of radiation including alpha radiation, beta radiation, gamma radiation. Examples include exposure to medical x-rays, PET scans, CT scans; nuclear radiation such as from nuclear fallout or nuclear accidents, UV radiation from sun exposure or other UV sources, cosmic radiation from extended time at high altitudes (e.g. pilots and aeroplane attendants, frequent aeroplane travelers, astronauts), and medical radiotherapy. For example, the nanoparticles can be used to treat radiation-induced lung fibrosis.

It will be appreciated that nanoparticles according to the invention may be used in a medicament, which may be a monotherapy (i.e. the sole use of that antagonist or medicament), for eliminating damaged and/or senescent cells or for treating, ameliorating or preventing a disease characterized by the presence of damaged and/or senescent cells.

Alternatively, nanoparticles according to the invention may be used as an adjunct to, or in combination with, known therapies for eliminating damaged and/or senescent cells or for treating, ameliorating or preventing a disease characterized by the presence of damaged and/or senescent cells. For example, the nanoparticles may be used in combination with known agents for treating pulmonary fibrosis, such as corticosteroids.

There is no restriction on which nanoparticle as described herein should be administered to which patient. Rather, it is intended that any of the nanoparticles described herein can be administered to any patient as described herein. It is expressly intended by the inventors, in fact, that each and every combination of nanoparticle, and indicated patient group, is encompassed by this invention. The invention thus includes each and every possible combination of nanoparticle and indicated patient group.

The nanoparticles according to the invention may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising nanoparticles according to the invention may be used in a number of ways. For instance, oral administration may be required, in which case the nanoparticles may be contained within a composition that may, for example, be ingested orally in the form of a tablet, capsule or liquid. Compositions comprising nanoparticles of the invention may be administered by inhalation (for example, intranasally). Compositions may also be formulated for topical use. For instance, creams or ointments may be applied to the skin, for example, adjacent the treatment site.

Nanoparticles according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with nanoparticles used according to the invention is required and which would normally require frequent administration (for example, at least daily injection).

In a preferred embodiment, nanoparticles and compositions according to the invention may be administered to a subject by injection into the blood stream or directly into a site requiring treatment. Injections may be intravenous (bolus or infusion) or subcutaneous (bolus or infusion), or intradermal (bolus or infusion).

It will be appreciated that the amount of nanoparticle that is required is determined by its biological activity and bioavailability, which in turn depends on the mode of administration, the physiochemical properties of antagonist, and whether it is being used as a monotherapy or in a combined therapy. The frequency of administration will also be influenced by the half-life of the nanoparticle within the subject being treated. Optimal dosages to be administered may be determined by those skilled in the art, and will vary with the particular nanoparticle in use, the strength of the pharmaceutical composition, the mode of administration, and the advancement of the disease or condition being treated. Additional factors depending on the particular subject being treated will result in a need to adjust dosages, including subject age, weight, gender, diet and time of administration.

Generally, a daily dose of between 10 mg/kg of body weight and 100 mg/kg of body weight of the nanoparticle according to the invention may be used for eliminating damaged and/or senescent cells or for treating, ameliorating or preventing a disease characterized by the presence of damaged and/or senescent cells, depending upon which antagonist is used.

More preferably, the daily dose is between 20mg/kg of body weight and 70mg/kg of body weight, more preferably between 30mg/kg and 50mg/kg body weight, and most preferably between approximately 30mg/kg and 40mg/kg body weight.

Generally, 100 mg of drug are encapsulated per 1g of nanoparticles and, upon digestion in *vitro,* approximately 30mg of drug are released per g of nanoparticles. Normally a dose of 1mg/kg of deliverable drug is used, which means administering approximately 33mg/kg of nanoparticles.

The nanoparticles may be administered before, during or after onset of the condition or disease characterized by the presence of damaged and/or senescent cells. Daily doses may be given as a single administration (for example, a single daily injection). Alternatively, the nanoparticles may require administration twice or more times during a day. As an example, nanoparticles may be administered as two (or more, depending upon the severity of the condition being treated) daily doses of between 2000mg and 3000mg (i.e. assuming a body weight of 70 kg). A patient receiving treatment may take a first dose upon waking and then a second dose in the evening (if on a two-dose regime) or at 3- or 4-hourly intervals thereafter.

Alternatively, a slow release device may be used to provide optimal doses of nanoparticles according to the invention to a patient without the need to administer repeated doses.

Known procedures, such as those conventionally employed by the pharmaceutical industry (for example, in *vivo* experimentation, clinical trials, etc.), may be used to form specific formulations comprising the nanoparticles according to the invention and precise therapeutic regimes (such as daily doses of the antagonists and the frequency of administration). The inventors believe that they are the first to describe a composition for treating disease characterized by the presence of damaged and/or senescent cells based on the use of the nanoparticles of the invention.

Thus, in a ninth aspect of the present invention, there is provided the nanoparticle according to the first aspect or the cell according to the fourth aspect, for use in cosmetics.

Accordingly, the nanoparticles can be applied topically to cosmetically improve the skin, for example, the removal of moles, which contain melanocytic nevi that have undergone senescence. For its application in altering cosmetic appearance, the products of the present invention will be provided, preferably, in a cosmetic formulation with a dermatologically acceptable carrier.

A "dermatologically acceptable carrier" is a carrier which is suitable for topical application to the skin. Suitable dermatologically acceptable carriers include: emulsions such as oil-in-water, water-in-oil, oil- in-water-in silicone and water-in-oil-in water emulsions; anhydrous liquid solvents such as oils (e.g. mineral oil), alcohols (e.g. ethanol, isopropanol), silicones (e.g. dimethicone, cyclomethicone); aqueous-based single phase liquid solvents.

In addition, the cosmetic formulation may comprise skin actives such as vitamins (e.g. vitamin B3, vitamin E, etc), hydroxy acids (e.g. salicylic acid, glycolic acid); exfoliation agents such as zwitterionic surfactants; sunscreen (e.g. 2-ethylhexyl-p- methoxycinnamate, 4,4'-t-butyl methoxydibenzoyl-methane, octocrylene, , zinc oxide, titanium dioxide); antiinflammatory agents; anti-oxidants (e.g. tocopherol); metal chelators (e.g. iron chelators); retinoids; depilatory agents; skin lightening agents; anti-microbial agents. In some embodiments, the cosmetic formulations may be in the form of ointments, pastes, creams, lotions, gels, powders, solutions or patches.

In certain embodiments, the formulations and compositions are creams, which may further contain saturated or unsaturated fatty acids such as steaeric acid, palmitic acid, oleic acid, palmato-oleic acid, acetyle, or aryl oleyl alcohols, stearic acid. Creams may also contain a non-ionic surfactant, for example, polyoxy-40-stearate.

In a tenth aspect of the invention, there is provided a damaged and/or senescent cells-treatment composition comprising a nanoparticle according to the first aspect or a cell according to the fourth aspect, and a pharmaceutically acceptable vehicle.

The phrase "damaged and/or senescent cells-treatment composition" can mean a pharmaceutical formulation used in the therapeutic amelioration, prevention or treatment of any disease or condition characterized by the presence of damaged and/or senescent cells in a subject.

In an eleventh aspect of the invention, there is provide a process for making the a damaged and/or senescent cells-treatment composition according to the tenth aspect, the process comprising contacting a therapeutically acceptable amount of a nanoparticle according to the first aspect or a cell according to the fourth aspect, and a pharmaceutically acceptable vehicle.

A "therapeutically effective amount" of a nanoparticle of the invention is any amount which, when administered to a subject, is the amount of nanoparticle that is needed to eliminate damaged and/or senescent cells, or produce the desired effect.

For example, the therapeutically effective amount of nanoparticle used may be from about 0.001 mg to about 1000 mg, and preferably from about 0.01 mg to about 500 mg. It is preferred that the amount of agent is an amount from about 0.1 mg to about 100 mg, and most preferably from about 0.5 mg to about 50 mg.

A "pharmaceutically acceptable vehicle" as referred to herein, is any known compound or combination of known compounds that are known to those skilled in the art to be useful in formulating pharmaceutical compositions.

A "subject", as used herein, may be a vertebrate, mammal or domestic animal. Hence, compositions and medicaments according to the invention may be used to treat any mammal, for example livestock (for example, cattle), pets, or may be used in other veterinary applications. Most preferably, though, the subject is a human being.

In one embodiment, the pharmaceutically acceptable vehicle may be a solid, and the composition may be in the form of a powder or tablet. A solid pharmaceutically acceptable vehicle may include one or more substances which may also act as flavoring agents, lubricants, solubilisers, suspending agents, dyes, fillers, glidants, compression aids, inert binders, sweeteners, preservatives, dyes, coatings or tablet-disintegrating agents. The vehicle may also be an encapsulating material. In powders, the vehicle is a finely divided solid that is in admixture with the finely divided active agents according to the invention. In tablets, the active agent may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The active agent according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavoring agents, suspending agents, thickening agents, colours, viscosity regulators, stabilisers or osmoregulators. Suitable examples of liquid vehicles for oral and parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurised compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The nanoparticle may be prepared as a sterile solid composition that may be dissolved or suspended at the time of administration using sterile water, saline or other appropriate sterile injectable medium.

The nanoparticles and compositions of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The nanoparticles used according to the invention can also be administered orally either in liquid or solid composition form. Compositions suitable for oral administration include solid forms, such as pills, capsules, granules, tablets and powders, and liquid forms, such as solutions, syrups, elixirs and suspensions. Forms useful for parenteral administration include sterile solutions, emulsions and suspensions.

It will be appreciated that the invention extends to any nucleic acid or peptide or variant, derivative or analogue thereof, which comprises substantially the amino acid or nucleic acid sequences of any of the sequences referred to herein, including functional variants or functional fragments thereof. The terms "substantially the amino acid/nucleotide/peptide sequence", "functional variant" and "functional fragment", can be a sequence that has at least 40% sequence identity with the amino acid/nucleotide/peptide sequences of any one of the sequences referred to herein, for example 40% identity with the sequences referred to herein.

Amino acid/nucleotide/peptide sequences with a sequence identity which is greater than 50%, more preferably greater than 65%, 70%, 75%, and still more preferably greater than 80% sequence identity to any of the sequences referred to herein are also envisaged.

Preferably, the amino acid/nucleotide/peptide sequence has at least 85% identity with any of the sequences referred to, more preferably at least 90%, 92%, 95%, 97%, 98%, and most preferably at least 99% identity with any of the sequences referred to herein.

The skilled technician will appreciate how to calculate the percentage identity between two amino acid/nucleotide/peptide sequences. In order to calculate the percentage identity between two amino acid/nucleotide/peptide sequences, an alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local *versus* global alignment, the pair-score matrix used (for example, BLOSUM62, PAM250, Gonnet etc.) and gap-penalty, for example, functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length-dependent. Therefore, the shorter a pair of sequences is, the higher the sequence identity one may expect to occur by chance.

Hence, it will be appreciated that the accurate alignment of amino acid or nucleic acid sequences is a complex process. The popular multiple alignment program ClustalW [48,49] is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/nucleotide/peptide sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)*100.

Alternative methods for identifying similar sequences will be known to those skilled in the art. For example, a substantially similar nucleotide sequence will be a sequence which hybridises to a nucleotide sequence encoding a peptide according to the first aspect, or a functional fragment or functional variant thereof, or their complements, under stringent conditions. By stringent conditions is meant that the nucleotide hybridises to filter-bound DNA or RNA in 3x sodium chloride/sodium citrate (SSC) at approximately 45 °C followed by at least one wash in 0.2x SSC/0.1% SDS at approximately 20-65 °C. Alternatively, a substantially similar peptide may differ by at least 1, but less than 3, 4, 5, 6, 7, 8, 9 or 10 amino acids from the sequences shown.

Due to the degeneracy of the genetic code, it is clear that any nucleic acid sequence described herein could be varied or changed without substantially affecting the sequence of the peptide, polypeptide or protein encoded thereby, to provide a functional variant thereof.

Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

According to a twelfth aspect of the invention there is provided a method of identifying damaged and/or senescent cells, the method comprising administering a nanoparticle according to the first aspect or a cell according to the fourth aspect and detecting the presence of damaged and/or senescent cells by detecting the presence of the payload.

In order to be able to identify damaged and/or senescent cells, the nanoparticle's payload may comprise a conventional contrast material that can be used to enhance the contrast of structures or fluids within the body during medical imaging. Such a material may be visible using Magnetic Resonance Imaging (MRI), Computed Tomography (CT) and/or Optical Imaging (OI).

Careful selection of the material used for the contrast material enables the nanoparticles to be used in either diagnosis and/or therapy of various diseases and conditions associated with the presence of damaged and/or senescent cells.

The contrast material, which is visible using MRI, CT or OI, may comprise a metallic or non-metallic material. The contrast material may comprise a magnetic or non-magnetic material.

In embodiments where the contrast material is magnetic, it may comprise an MRI contrast material. The contrast material may comprise a paramagnetic or superparamagnetic material. For example, the contrast material may comprise iron, nickel, cobalt or dysprosium or a compound, such as oxide or alloy, which contain one or more of these elements. Preferably, the contrast material comprise magnetite (Fe₃O₄).

In embodiments where the contrast material is non-magnetic, it may comprise a MRI, CT or OI contrast material. For example, the contrast material may comprise gadolinium, gold, iodine or boro-sulphate. Each of these materials may be used as either MRI, CT or OI contrast materials. Preferably, the contrast material comprises gadolinium.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying diagrammatic drawings, in which:

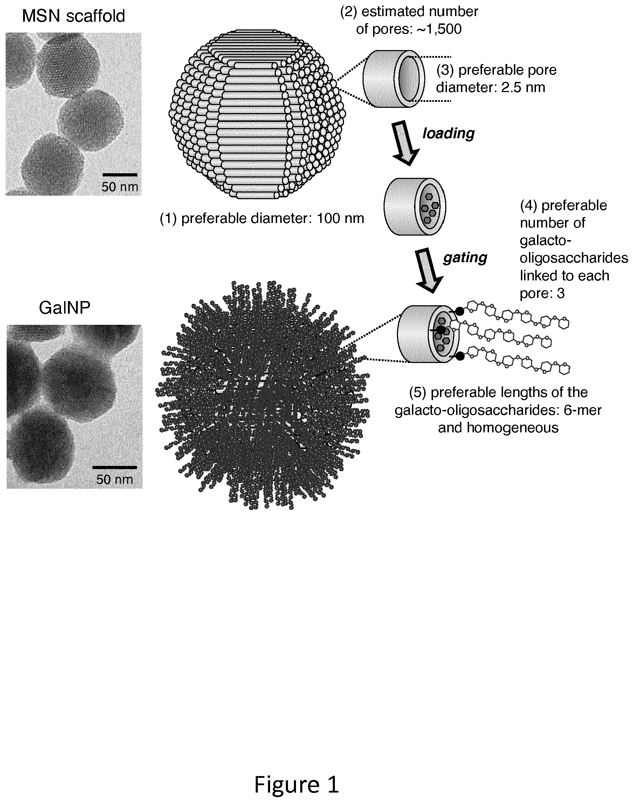

**Figure 1** shows Transmission Electron Microscopy (TEM) images of calcined MSNs (MSN scaffold) and GalNP(rho), showing the typical porosity of the MSNs matrix visualized as alternate black and white stripes. The prepared NPs-based materials are obtained as spherical particles with diameter ca. 100 nm. GosNP(rho), GosNP(icg), and GalNP(dox) show TEM images similar to this for GalNP(rho). The panel also shows a schematic drawing of the MSN scaffold and the GalNPs specifying: (1) the preferred diameter of the particle, (2) the estimated number of pores per particle, (3) the preferred diameter of the pores (cavities), (4) the preferred number of saccharides linked to each cavity to gate the pores, (5) the preferred length of the saccharides.

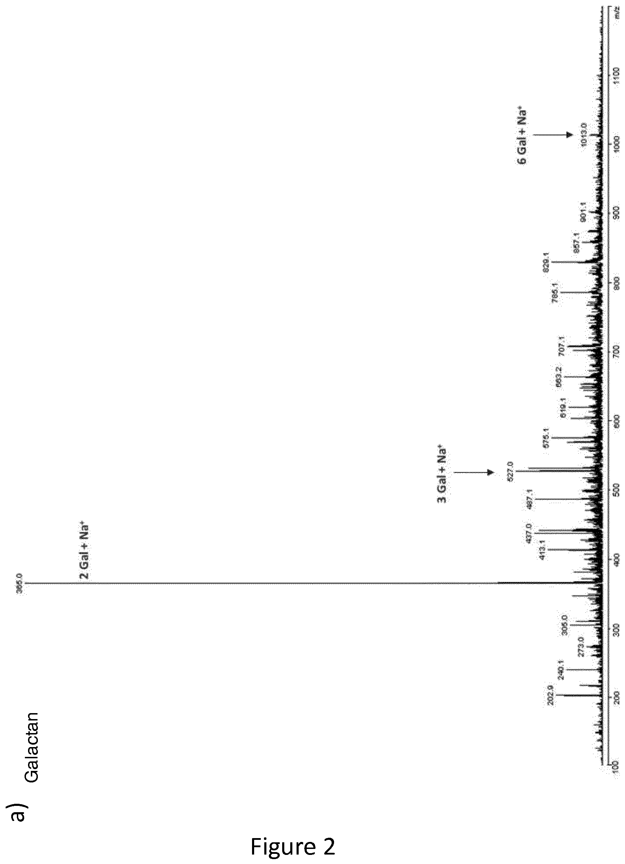

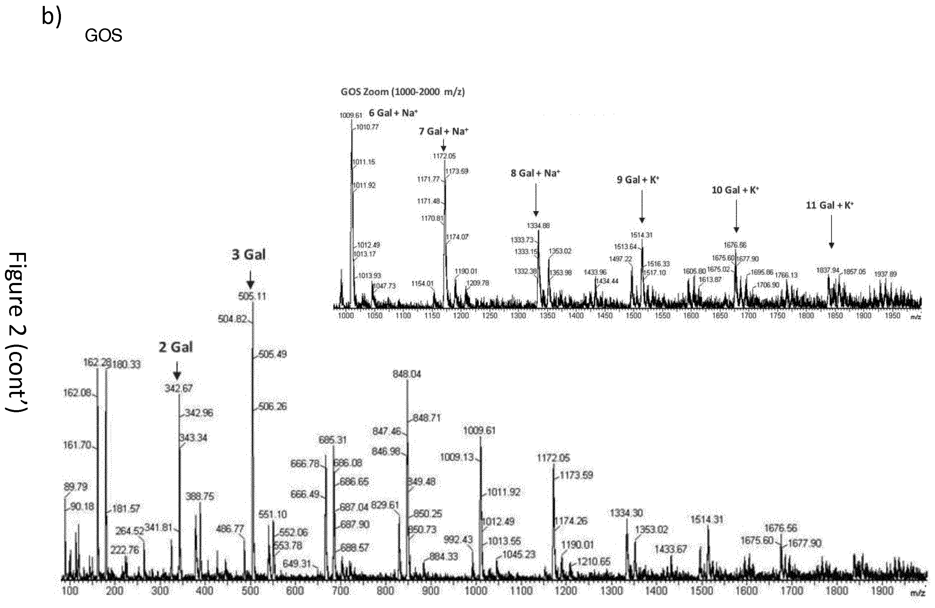

**Figure 2** shows in Panel a) Mass spectra of Galactan showing a molecular ion peak corresponding to six linked galactoses (1013 m/z = 990 g_{GALACTAN/}mol + 23 g_{Na}/mol), and fragmentations of trimer and dimer oligosaccharides (base peak). Panel b) shows Mass spectra of GOS, which is much more complex and shows several peaks. The molecular ion peak corresponds to eleven linked galactoses (1837 m/z = 1798 g_{GOS}/mol + 39 g_{K/}mol). Besides, peaks corresponding from 10 to 2 linked galactoses can be observed (base peak corresponds to trimer). Inset shows magnification of the graph from 1000 to 2000 m/z.

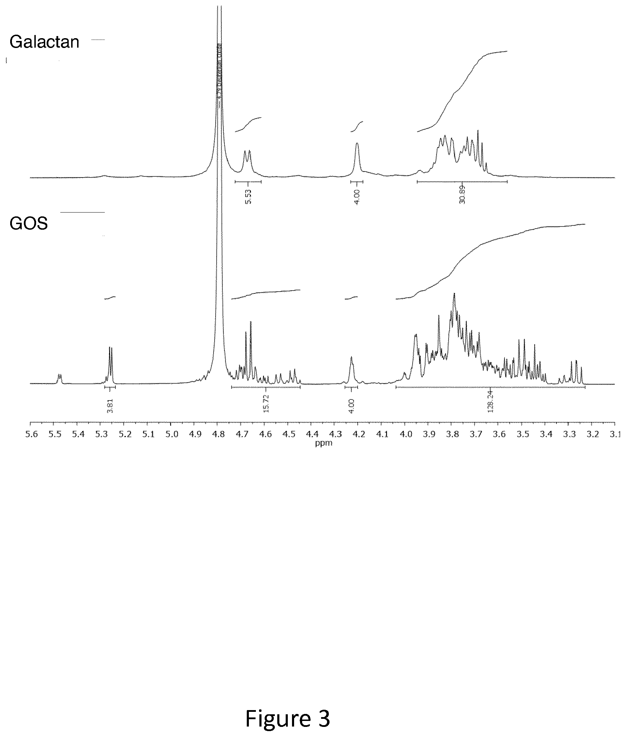

**Figure 3** presents ¹H-NMR spectra of Galactan and GOS. Both spectra present the same signal centered at 4.24 ppm. Considering that this signal corresponds to 4 protons for the Galactan spectra, the signal of the proton linked to anomeric carbon (centered at 4.68 ppm) correlates to 6 protons (5.53), indicating the presence of six anomeric carbons in the molecule of Galactan, supporting the presence of six galactoses in the molecule. Signals from 4 to 3.6 ppm interval, integrate approximately 30 protons (30.89) that correspond to protons in the Galactan molecule linked directly to C-OH. Protons of hydroxyl groups are labile and do not appear when D₂O is used as solvent. In the case of the GOS spectra, anomeric proton signals are highly complex and integrate more than 15 protons (15.72). The rest of signals integrate more than 100 protons (128.24), demonstrating that GOS is a complex mixture of polysaccharides, whereas Galactan is exclusively a hexamer of galactoses.

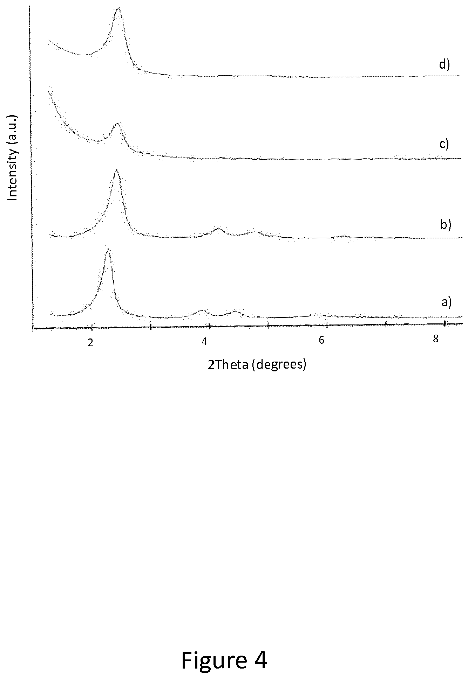

**Figure 4** presents powder X-ray diffraction patterns for a) MSNs as-synthesized, b) calcined MsNs, c) GosNP(rho), and d) GalNP(rho); the XRD of NPs as-synthesized (curve a) shows the typical four low angle reflections of a hexagonal ordered array indexed as (100), (110), (200), and (210) Bragg peaks. In curve b, corresponding to the NPs calcined sample, a significant shift of the (100) reflection in the XRD is clearly observed. This displacement, together with the broadening of the (110) and (200) reflections, is consistent with an approximate cell contraction of ca. 6-8 Å and attributed to the condensation of silanols during the calcination step. XRD patterns for solids GosNP(rho) and GalNP(rho) (curves c and d, respectively). For these materials, the reflections (110) and (200) are lost, most likely due to a reduction of contrast. Nevertheless, the presence of the d100 peak in the XRD patterns in all cases indicated that the process of pore loading and the additional functionalization with the corresponding saccharides, did not to a large extent modify the mesoporous scaffolding. GosNP(icg), and GalNP(dox) show XRD profiles similar to those from GosNP(rho) and GalNP(rho).

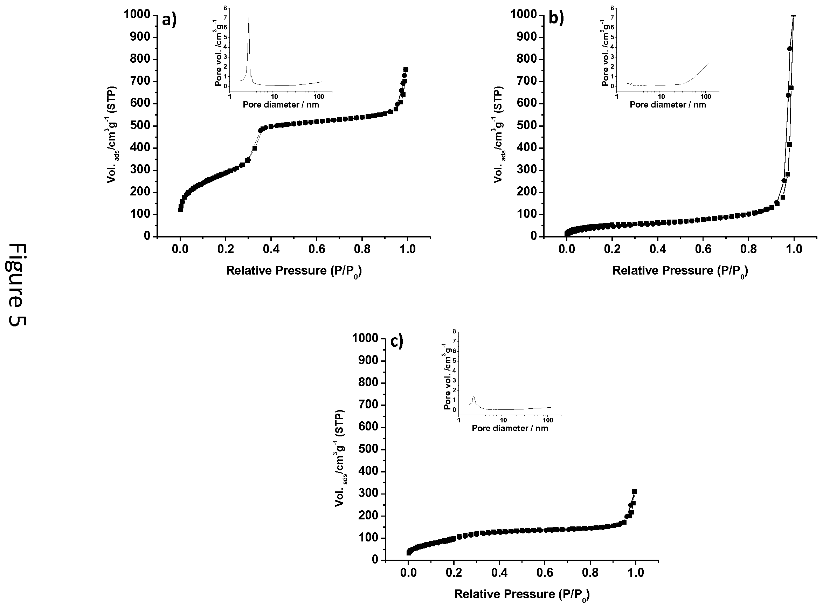

**Figure 5** shows nitrogen adsorption-desorption isotherms for a) MSN mesoporous material, b) GosNP(rho)n material, and c) GalNP(rho) material. Insets show pore size distribution of each material. A typical curve for these mesoporous solids consisting of an adsorption step at intermediate P/Po value (0.1-0.3) can be observed in panel a. This curve corresponds to a type IV isotherm, in which the observed step deals with nitrogen condensation inside the mesopores. The absence of a hysteresis loop in this interval and the narrow BJH pore distribution suggest the existence of uniform cylindrical mesopores (pore diameter of 2.51 nm and pore volume of 0.72 cm3 g-1 calculated by using the BJH model on the adsorption branch of the isotherm). The application of the BET model resulted in a value of 996 m2/g for the total specific surface. Panels b and c show nitrogen adsorption-desorption isotherm of GosNP(rho) and GalNP(rho), respectively. The isotherms are typical of mesoporous systems with practically filled mesopores. Consequently, relatively low N2 adsorbed volume (BJH mesopore volume = 0.436 cm3 g-1 and 0.418 cm3 g-1 respectively) and surface area (197 m2/g and 373 m2/g) values were calculated. These solids show flat curves when compared (at the same scale) to those of the NPs parent material, which indicates significant pore blocking and the subsequent absence of significant mesoporosity. GosNP(icg), and GalNP(dox) show similar N2 adsorption-desorption isotherms to that shown by GosNP(rho) and GalNP(rho).

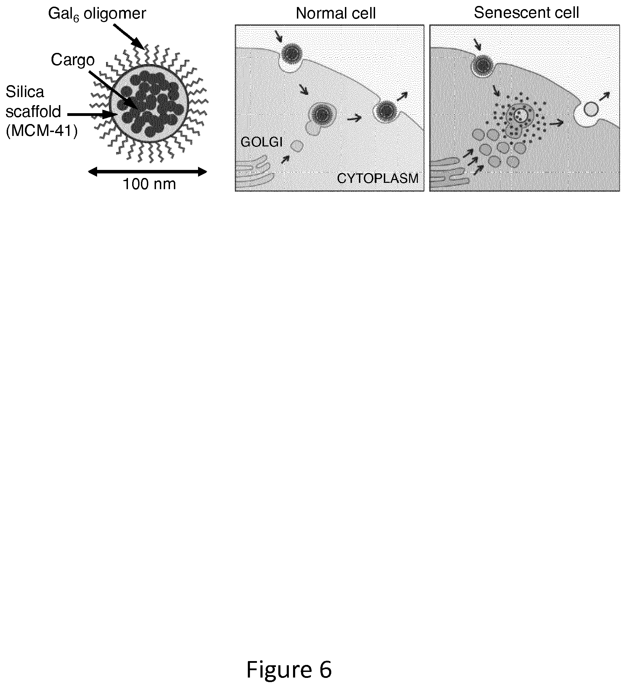

**Figure 6** shows a schematic representation of the GalNP beads, which are based on a mesoporous silica scaffold (MCM-41) that can be loaded with different cargoes encapsulated by a coat of 6-mer β (1,4)-galacto-oligosaccharides. Cellular uptake of the GalNP beads occurs via endocytosis and, after fusion with lysosomal vesicles, the beads are released by exocytosis. The high lysosomal β galactosidase activity of senescent cells allows a preferential release of the cargo by a β galactosidase-mediated hydrolysis of the cap..

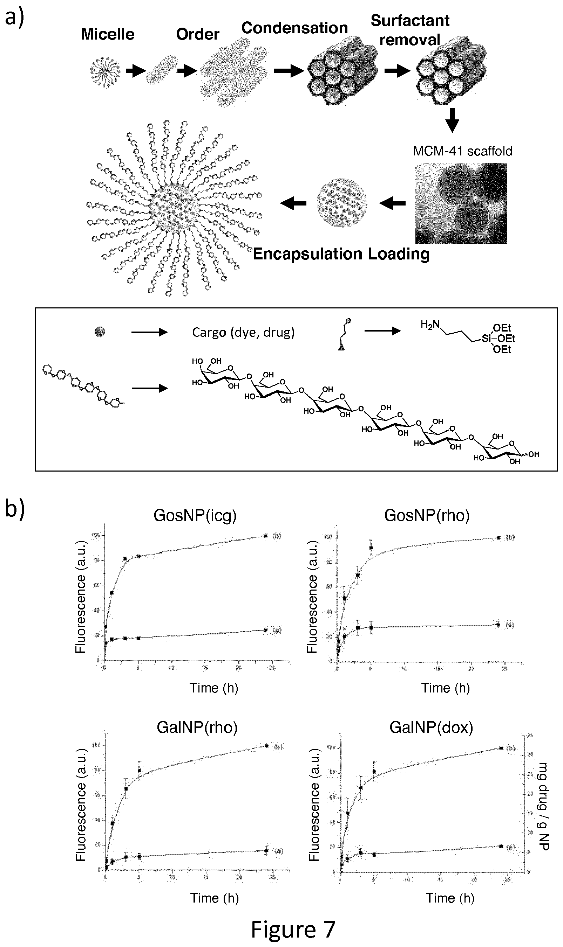

**Figure 7** shows in Panel a) a representation of the synthesis of the GalNP beads. First, a super-micellar template is formed in basic water solution. Next, the inorganic siliceous precursor tetraethylorthosilicate hydrolyses and condensates of around the template. The final mesoporous inorganic MCM-41 scaffold, which presents cylindrical unidirectional empty channels arranged in a hexagonal distribution, is obtained by the removal of the surfactant template by calcination. Next, the cargo is loaded in the mesopores and, finally, the beads are encapsulated with 6-mer galacto-oligosaccharides that will act as molecular gate to obtain the final GalNP system. Panel b) shows cargo release studies of the GosNP(rho), GosNP(icg), GalNP(rho), and GalNP(dox) beads by spectrofluorometry. The graphs represent the release profiles of the cargoes in the absence (a) or the presence (b) of β-galactosidase from *Aspergillus oryzae* in water at pH 4.5 at room temperature at the indicated time points..

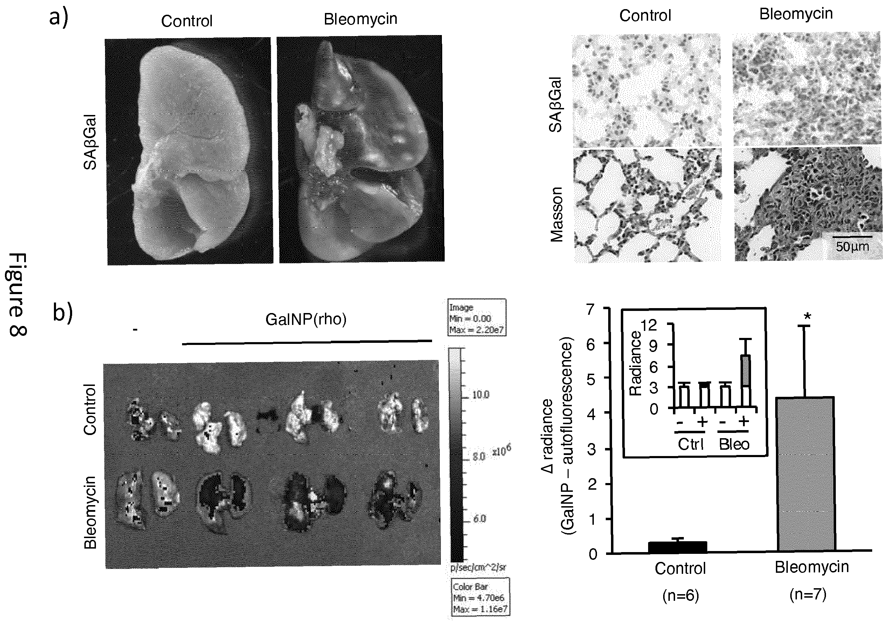

**Figure 8** shows in Panel a) C57BL/6 mice subjected to a single intratracheal administration bleomycin (1.5 U/kg) and analyzed 2 weeks later. Lungs were collected and stained (whole organ staining) for SAβGal. Pictures at the left correspond to representative lungs. The right panel shows sections of control and bleomycin-treated lungs processed for SAβGal and Masson's trichrome staining (collagen fibers appear in blue in lower panels), as indicated. In Panel b) control and bleomycin-treated mice, as in panel a), were tail vein injected with 200 µl of a solution containing GalNP(rho) (4 mg/ml). At 6 h post-injection, mice were sacrificed and the lungs were analyzed by an IVIS spectrum imaging system. The graph indicates the average difference in lung radiance between GalNP-injected and non-injected (autofluorescence) mice, for both control and bleomycin-treated groups. Values are expressed as mean ± SD, and statistical significance was assessed by the two-tailed Student's t test: *p < 0.05. The inset shows the absolute values of radiance (p/s/cm²/sr x 10⁶) for each group. The corresponding differences are highlighted.

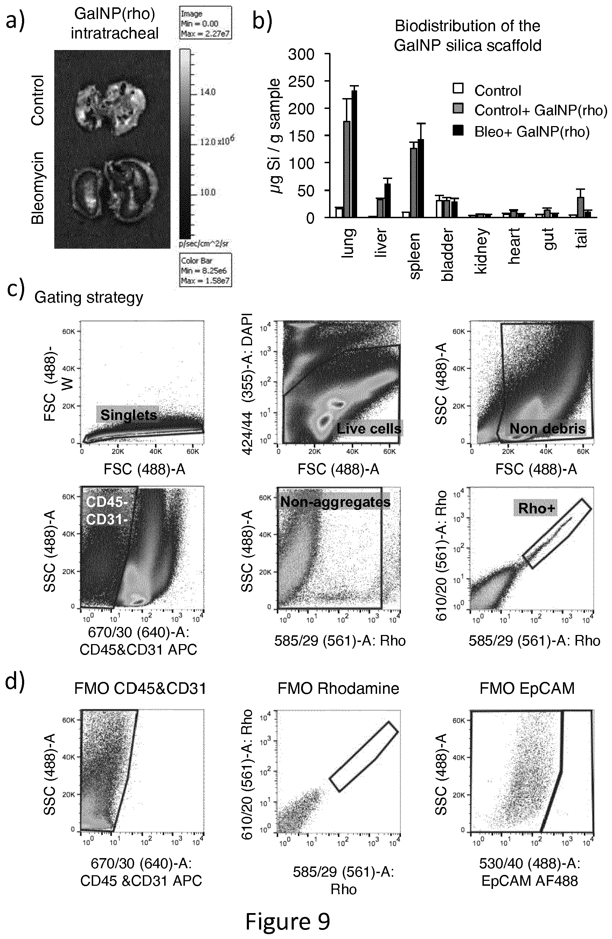

**Figure 9** shows in Panel a) C57BL/6 mice subjected to a single intratracheal administration of bleomycin (1.5 U/kg). 2 weeks later, mice were intratracheally injected with 30 µl of a solution containing GalNP(rho) (4 mg/ml). At 6 h post-inoculation, mice were sacrificed and the lungs were analyzed by an IVIS spectrum imaging system. In Panel b), biodistribution studies of the GalNP silica scaffold in lung, liver, spleen, bladder, kidney, heart, gut and tail. Control and bleomycin-treated C57BL/6 mice were tail vein injected with 200 µl of a solution containing GalNP(rho) (4 mg/ml). At 6 h post- GalNP(rho) injection the indicated organs were extracted for Si detection. The graph shows the µg Si per g of sample detected by Inductively Coupled Plasma Mass Spectroscopy (ICP-MS) in the indicated organs. In Panel c) gating strategy was used to exclude endothelial and haematopoietic cells. Cells were sorted using pulse processing to exclude cell aggregates; DAPI was used as dead discriminator; and CD45 and CD31 were used to exclude endothelial (CD31+) and haematopoietic cells (CD45+). Aggregates of GalNP beads were excluded from the analysis as indicated. In Panel d) fluorescence minus-one (FMO) controls of the indicated fluorophores.

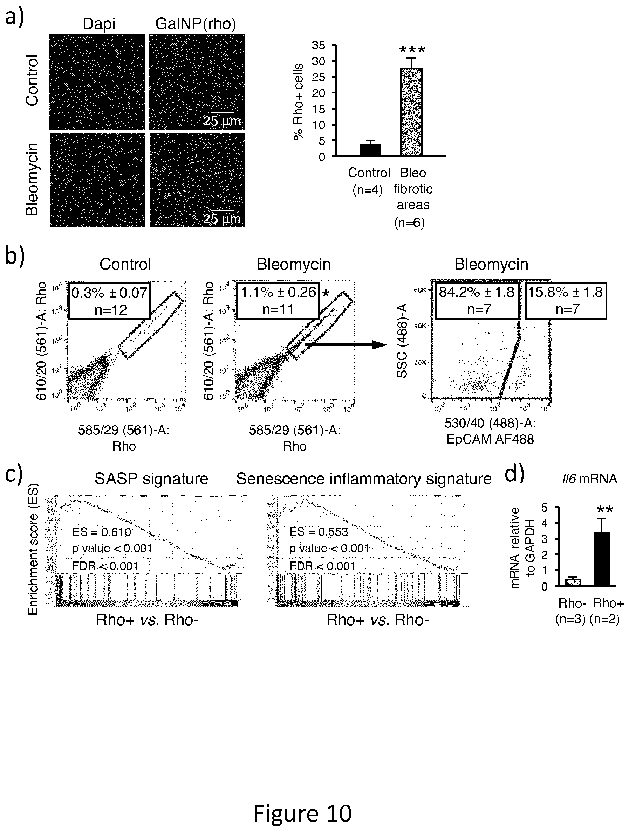

**Figure 10** shows in Panel a) control and bleomycin-treated mice (2 weeks post-bleomycin), were injected with GalNP(rho) (50 µg/ml) and lung sections were analyzed 6 h post-injection by confocal microscopy to detect rhodamine. Pictures correspond to representative sections of control and bleomycin-treated lungs. The graph shows % of rhodamine+ cells in fibrotic areas from bleomycin-treated mice compared to normal lung tissue from control mice. Values are expressed as mean ± SD, and statistical significance was assessed by the two-tailed Student's t test: ***p < 0.001. Panel b) shows lung cell suspensions from control and bleomycin-treated mice (2 weeks post-bleomycin)were analyzed by fluorescence-activated cell sorting (FACS) to isolate Rho+ cells (after exclusion of CD45+ and CD31+ cells). The two dot plots to the left are representative examples. Values in boxes correspond to the mean ± SEM, and statistical significance was assessed by the two-tailed Student's t test: *p < 0.05. The panel to the right shows a representative dot plot of rhodamine+ cells (after exclusion of CD45+ and CD31+ cells) separated in EpCAM- and EpCAM+ subpopulations, from bleomycin-treated lungs. Values in boxes correspond to the mean ± SEM of these two populations. Panel c) shows GSEA plots of published signatures of SASP and SIR (senescence-inflammatory response) (Lasry and Ben-Neriah, 2015) against the ranked list of differential expression between Rho+ and Rho- cells (all CD45-CD31-) from bleomycin-treated mice (n=3), at 2 weeks post-bleomycin. Panel d) shows levels of Il6 mRNA measured by quantitative PCR in the indicated lung cell populations (Rho+ or Rho-, all CD45-CD31-) from mice treated with bleomycin as in the above panels. Gapdh was used for input normalization. Values are expressed as mean ± SD, and statistical significance was assessed by the two-tailed Student's t test: **p < 0.01.

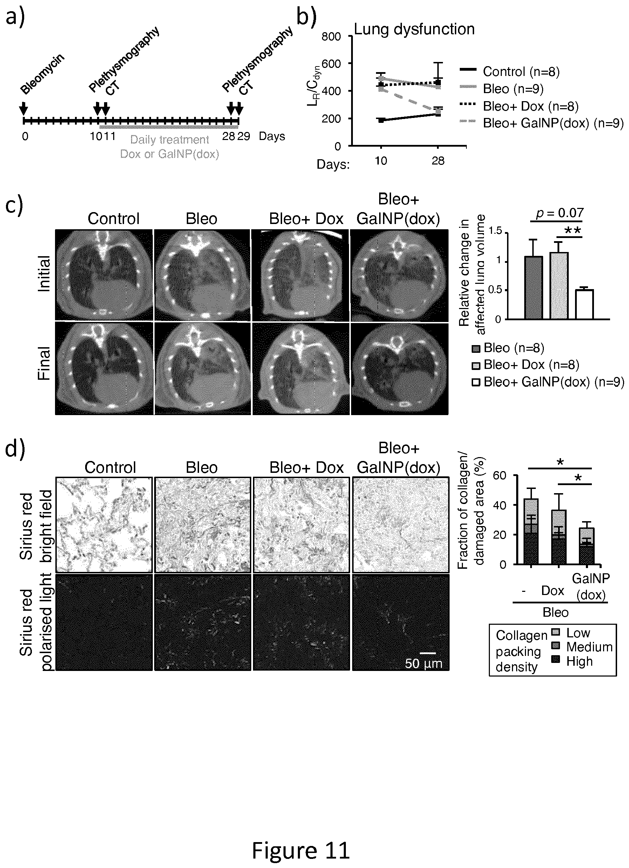

**Figure 11** shows in Panel a) C57BL/6 male mice were subjected to a single intratracheal administration of bleomycin at 1.5 U/kg BW. Beginning at day 10, mice were treated daily with free doxorubicin (tail vein injection, 1 mg/kg) or with GalNP(dox) (tail vein injection, 200 µl of a solution with 4 mg/ml of GalNP containing a total of 1 mg/kg of deliverable doxorubicin), for 18 days, that is, until day 28 post-bleomycin. Plethysmography and CT were performed at the indicated days. Panel b) shows plethysmography was used to determine the ratio between lung resistance (LR) and compliance dynamics (Cdyn) in the indicated groups before and after the indicated treatments. Values are expressed as mean ± SEM. Panel c) shows representative CT images of the indicated treatments at days 11 and 29 post bleomycin injury. The graph represents the fold change in affected lung volume after the indicated treatments. Values are expressed as mean ± SEM, and statistical significance was assessed by the two-tailed Student's t test: **p < 0.01. Panel d) shows representative images of Sirius Red staining of lungs from mice subjected to the indicated treatments two days after the last CT analysis, visualised by bright field or polarised light. The graph represents the fraction of collagen per damaged area, showing densely, intermediately or loosely packed collagen. Values are expressed as mean ± SD, and statistical significance was assessed by the two-tailed Student's t test: *p < 0.05.

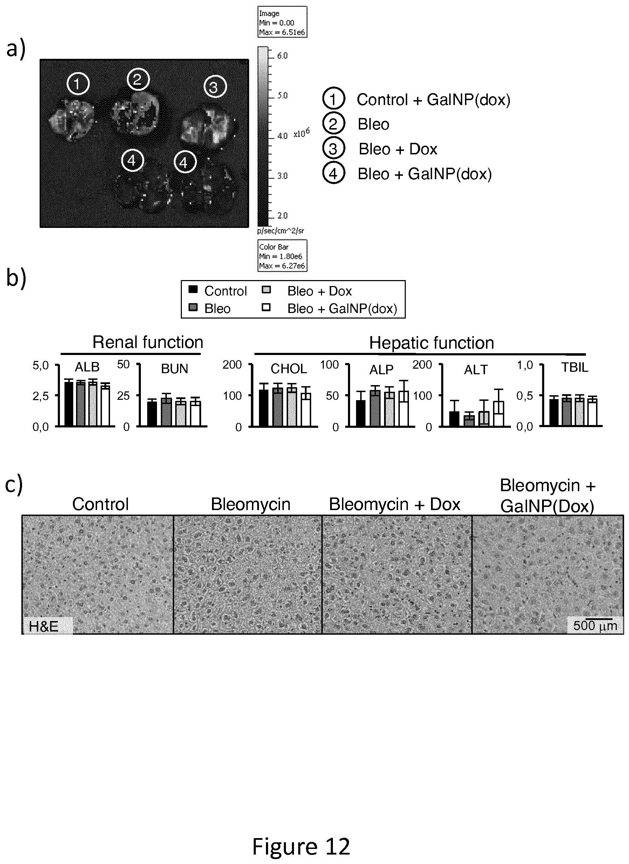

**Figure 12** shows in Panel a) C57BL/6 male mice were subjected to a single intratracheal administration of bleomycin at 1.5 U/kg BW. Beginning at day 14, mice were treated every four days with free doxorubicin (tail vein injection, 4 mg/kg) or every two days with GalNP(dox) (tail vein injection, 200 µl of a solution with 4 mg/ml of GalNP containing a total of 1 mg/kg of deliverable doxorubicin) for 14 days, that is, until day 28 post-bleomycin. At the end of the treatment mice were sacrificed, lungs were collected, and doxorubicin fluorescence was analyzed by an IVIS spectrum imaging system. Panel b) shows analysis of renal and hepatic function of mice as in Fig. 11 at the end of the treatment. ALB, albumin; BUN, blood urea nitrogen; CHOL, cholesterol; ALP, alkaline phosphatase; ALT, alanine transaminase; TBIL, total bilirubin. Panel c) shows haematoxylin and eosin (HE) staining of the livers of mice as in Fig. 14 at the end of the treatment.

**Table 1. BET specific surface values, pore volumes and pore sizes calculated from the N2 adsorption-desorption isotherms for selected materials.**

| | SBET (m² g⁻¹) | Pore Volume ^{a} (m² g⁻¹) | Pore size ^{a,b} (nm) |
|---|---|---|---|
| NPs | 996 | 0.720 | 2.51 |
| GosNP(rho) | 197 | 0.436 | --- |
| GalNP(rho) | 372 | 0.418 | --- |

| | | | |
|---|---|---|---|
| ^{a}) Pore volumes and pore sizes are only associated with intraparticle mesopores. ^{b}) Pore size estimated by using the BJH model applied on the adsorption branch of the isotherm. | | | |

**Table 2. Content (α) in mg of 'saccharide' and cargo for solids GosNP(rho), GosNP(icg), GalNP(rho), and GalNP(dox).**

| Solid | α saccharide (mg/g solid) | α cargo (mg/g solid) |
|---|---|---|
| GosNP(rho) | 141.7 | 97.80 |
| GosNP(icg) | 151.4 | 43.30 |
| GalNP(rho) | 137.8 | 147.9 |
| GalNP(dox) | 192.1 | 101.3 |

**Table 3. Amount (in mg (α) or as percentage (%)) of maximum drug release for solids GalNP(dox).**

| Solid | α drug (mg/g solid) | % of loaded drug that is released□ |
|---|---|---|
| GalNP(dox) | 31.05 | 30.65 |

### Examples

The invention will now be described by way of illustration only in the following examples.

The materials and methods employed in the studies described in the Examples were as follows, unless where otherwise indicated:

### Materials and Methods

### Synthesis of nanoparticles

For the synthesis of the mesoporous silica nanoparticles (MSNs) scaffolds, N-cetyltrimethylammonium bromide (CTAB, Sigma, #H6269) was first dissolved in 480 ml of deionized water at 2.74 mM. Then, 3.5 mL of NaOH 2 M (Sigma, #1310732) were added to the CTAB solution, and the temperature adjusted to 80°C. Next, 5 ml of the polymeric precursor tetraethylorthosilicate (TEOS, Sigma, #131903) was added dropwise to the surfactant solution. The mixture was stirred for 2 h at 80°C, until a white precipitate, referred to as "solid product", was obtained. This solid product was centrifuged at 10,000 rpm for 20 min and was washed with deionized water until a neutral pH was reached. It was then dried at 60°C, after which it was referred to as "MSNs as synthesized". To obtain the final template-free MSN scaffolds, the "MSNs as synthesized" were calcined at 550°C using an oxidant atmosphere for 5 h to remove the CTAB template, thus eliminating any residual surfactant that might affect cell viability, and thereby obtaining the "MSN scaffolds", see Figure 7a.

Loading of the MSN scaffolds with the different compounds was as follows: for NP(rho), 200 mg of MSN scaffolds were suspended in a solution of 16 ml of EtOH together with 76.65 mg of Rhodamine B (Sigma, #R6226); for NP(ICG), 200 mg of MSN scaffolds were suspended in a solution of 5 ml of water together with 5 mg of indocyanine green (ICG, Sigma); for NP(dox), 200 mg of MSN scaffolds were suspended in a solution of 12.5 ml of water together with 110 mg of doxorubicin (Carbosynth, #AD15377). These solutions were stirred in roundbottom flasks for 24 h at room temperature, and solids were isolated by centrifugation at 10,000 rpm for 15 min, and dried at 37°C. This product was referred to as "loaded MSNs".

To coat them with galacto-hexasaccharides, the loaded MSNs were suspended in 7.5 ml of dichloromethane, and 280 µl of 3-aminopropyltriethoxysilane (APTES, Sigma, #440140) was added to the reaction mixture. After stirring for 5.5 h, solids were isolated by centrifugation at 10,000 rpm for 15 min and dried at 37°C. The obtained solids were suspended in 24 ml of water and 383 mg of Galactan (Carbosynth, #OG71532), mostly (≥80%) consisting of six repeating galactose monosaccharides linked through b-1,4 glycosidic bonds. Stirring was continued at room temperature for 21 h. The final product, referred to as "GalNP(cargo)", was obtained after several washes with water (cycles of centrifugation at 10,000 rpm for 15 min and suspension in water), followed by drying at 37°C. The GalNP(cargo) were then stored in a desiccator at room temperature", see Figure 7a.

In a few cases, a mixture of galacto-oligosaccharides (GOS) of different lengths (mostly dimers and trimers) was used to coat the "loaded MSNs", as previously reported (Agostini *et al.,* 2012). The galacto-oligosaccharide consists in a heterogeneous mixture of at least 60% of galacto-oligosaccharides; 33% disaccharides, 39% trisaccharides, 18% tetrasaccharides, 7% pentasaccharides and 3% of higher saccharides. The galacto-oligosaccharide was provided was provided by Zeus Quimica (Spain) as a syrup with a pH 3.8. This syrup was diluted in water and the pH was increased with NaHCO₃ up to 7. Then, the solution was lyophilized giving a white solid. Afterwards, 5 g of this solid were dissolved in anhydrous ethanol (total volume 100 mL) and a solution of 3-aminopropyltriethoxysilane (2, 5.85 mL, 25 mmol) was added. The reaction mixture was stirred for 24 h at room temperature to generate the galacto-oligosaccharide derivative (GOS-d). The solvent was evaporated under reduced pressure to give a white solid. Subsequently, 600 mg of galacto-oligosaccharide derivative (GOS-d) were dissolved in 20 ml of EtOH and added to the "loaded MSNs". The suspension was stirred for 5.5 h at room temperature. The final solids were obtained by centrifugation at 10,000 rpm, for 15 min, and were washed several times with water to eliminate non-anchored molecules and non-loaded dye. Finally, solids were dried at 37°C to obtain the final GOS-coated loaded MSNs (abbreviated as "GosNP(cargo)") ", see Figure 7a.

### Characterisation of nanoparticles

To characterize the nanomaterials, powder X-ray diffraction (XRD), transmission electron microscopy (TEM), N₂ adsorption-desorption, thermogravimetric analysis (TGA), elemental analysis (EA), fluorescence spectroscopy and UV-visible spectroscopy techniques were used.

The following equipment was used: X-ray measurements were performed on a Bruker AXS D8 Advance diffractometer using Cu-Kα radiation, Figure 4. TEM images were taken with a Philips CM10 microscope working at 100 kV, see Figure 1. N2 adsorption-desorption isotherms were recorded on a Micromeritics ASAP2010 automated sorption analyser. The samples were degassed at 120°C under vacuum overnight, see Figure 5. The specific surface areas were calculated from the adsorption data in the low-pressure range using the Brunauer-Emmett-Teller (BET) model. Pore size was determined by following the Barrett-Joyner-Halenda (BJH) method. The values obtained for these three features are shown in Table 1. Thermogravimetric analysis was carried out on TGA/SDTA 851e Mettler Toledo equipment, using an oxidant atmosphere (air, 80 mL/min) with a heating program consisting of a heating rate of 10°C per minute from 393 K to 1273 K and an isothermal heating step at this temperature for 30 min, values obtained with TGA are shown in Table 2. UV-visible spectroscopy was carried out with a Lambda 35 UV/vis spectrometer (Perkin-Elmer Instruments), and fluorescence spectroscopy was performed with a JASCO spectrofluorometer FP-8300.

¹H and ¹³C NMR spectra were recorded on a Bruker FT-NMR Avance 400 (Ettlingen, Germany) spectrometer at 300K, using tetramethylsilane (TMS) as an internal standard, Figure 3. Deuterium oxide (D₂O) was purchased from Sigma-Aldrich. GOS was purchased from NFBC King-Prebiotics (GOS-570-S) and lyophilized. Samples for NMR studies were prepared by dissolving 10 mg of product in 700µl of D₂O. Mass spectrometry (MS) data were obtained with a TRIPLETOF T5600 (ABSciex, USA) spectrometer. Samples for MS studies were prepared by dissolving 0.5 mg of product in 1ml of distilled water, Figure 2.

### Nanoparticles cargo release studies

2 mg of GosNP(rho), GosNP(ICG), GalNP(rho), and GalNP(dox) nanoparticles were suspended in 5mL of water at pH 4.5, and *β*-galactosidase from *Aspergillus oryzae* (Sigma) was added at 1,000 ppm. Suspensions were stirred and different aliquots were taken and centrifuged at different time points. The amount of cargo released was determined in a JASCO spectrofluorometer FP-8300 by monitoring the emission (for rho, ICG, dox) or the absorptionof the dye or drug in the aqueous solution, as a function of time (rho λₑₓ = 550 nm, rho λₑₘ = 580 nm; ICG λₑₓ = 775 nm, ICG λₑₘ = 799 nm; dox λₑₓ = 495 nm, dox λₑₘ = 556 nm), Figure 7b. The amount (in mg (α) or as percentage (%)) of maximum drug release for solid GalNP(dox) is reported in Table 3. The same procedure was performed without adding the enzyme to the nanoparticle suspension, as a "blank" control.

For cargo release imaging in lung cells, control and bleomycin-treated mice were tail vein injected with 200 µl of a solution containing GalNP(rho) at 4 mg/ml in DMEM. Mice were sacrificed 6 h post-injection, and lungs were placed in a cryomold containing OCT (Leica Biosystems) and frozen in dry ice for 30 min. Frozen OCT sections were washed in PBS and fixed in fresh 4% paraformaldehyde for 5 min at room temperature. Coverslips were mounted by using Vectashield Mounting Medium supplemented with 1.5 mg/ml DAPI prior to imaging.

Images were acquired on a TCS-SP₅ (AOBS) laser scanning confocal microscope (Leica Microsystems) using a 20X HCX PL APO 0.7 NA dry objective or a 40X HCX PL APO 1.2 NA oil immersion objective. Rhodamine B and doxorubicin were detected by using excitation wavelength of 561 nm (DPSS laser) and with a detection window between 570 and 590 nm. Auto-fluorescence was detected by using an excitation wavelength of 488 nm (Argon laser) and with a detection window between 495 and 535 nm. Images were analysed with LAS AF v2.6 acquisition software equipped with HCS-A module (Leica Microsystems). Rhodamine B intensity relative to cell surface was quantified with Definiens XD Developer v2.5 software (Definiens).

### Immunohistochemistry on paraffin sections

Tissue samples were fixed in 10% neutral buffered formalin (4% formaldehyde in solution), paraffin-embedded and cut in 3 mm sections, which were mounted and dried. For different staining methods slides were deparaffinised in xylene and re-hydrated through a series of graded EtOH until water. Masson's trichrome and Sirius red stainings were used to assess the presence of fibrotic areas in the lungs, as indicated. Haematoxylin and eosin (HE) staining was used to analyse liver damage, as indicated. For immunohistochemistry, an automated immunostaining platform was used (Ventana discoveryXT, Roche). Antigen retrieval was first performed with high pH buffer (CC1m, Roche), endogenous peroxidase was blocked and slides were then incubated with the appropriate primary antibodies as detailed: Ki67 (Master Diagnostica, #0003110QD), phosphorylated Rb (Ser807/811) (Cell Signalling Technology, #9308). After incubations with the primary antibody, slides were incubated with the corresponding secondary antibodies and visualization systems (OmniRabbit, Ventana, Roche) conjugated with horseradish peroxidase (Chromomap, Ventana, Roche). Immunohistochemical reactions were developed by using 3,30-diaminobenzidine tetrahydrochloride (DAB) as a chromogen, and nuclei were counterstained with hematoxylin. Finally, the slides were dehydrated, cleared and mounted with a permanent mounting medium for microscopic evaluation.

### Mice

All mice were maintained at the Spanish National Cancer Research Centre (CNIO) under specific pathogen-free conditions in accordance with the recommendations of the Federation of European Laboratory Animal Science Associations (FELASA). All animal experiments were approved by the Ethical Committee for Research and Animal Welfare (CEIyBA) and performed in accordance with the guidelines stated in the International Guiding Principles for Biomedical Research Involving Animals, developed by the Council for International Organizations of Medical Sciences (CIOMS). C57BL/6 mice were bred in the CNIO Animal Facility. Athymic nude mice (Hsd:Athymic Nude-Foxn1nu) were purchased from Envigo.

### Fibrosis

To induce pulmonary fibrosis, 8- to 10-week-old C57BL/6 wild-type mice were anesthetised by intraperitoneal injection with a mixture containing Ketamine (75mg/kg) and Medetomidine (1mg/kg). The animals were placed on a Tilting WorkStand for rodents (EMC Hallowell) and intubated intratracheally with a 24GA catheter (BD Biosciences). Then, bleomycin (Sigma, #15361) was intratracheally inoculated in at 1.5 U/kg of body weight. Serial sections of the lung were stained with Sirius red to assess the presence of collagen. Whole slides were digitalized with a slide scanner (Mirax Scan, Zeiss) using polarized light and images were acquired with the Pannoramic Viewer Software (3DHISTECH). Image analysis and quantification of coloured signals (red, yellow and green) within damaged areas was performed in a completely automated manner using the AxioVision software package (Zeiss). At least 7 mice per group were quantified.

### Treatments of the mice

In vitro experimental determinations indicated that approximately ∼30 mg of drug, doxorubicin is deliverable per g of nanoparticle (see Table 3). Nanoparticles were weighted in 5ml glass vials and stirred in DMEM supplemented with 10% FBS for 1h at 4 mg/ml. Mice were intravenously (i.v.) injected as indicated, with 200 µl of nanoparticle solution (equivalent to 1 mg/kg of releasable drug). Doxorubicin (Sigma, #D1515) dissolved in 7% saline/DMEM solution and administered by daily i.v. injection as indicated.

### Flow cytometry

Mice were anesthetised by intraperitoneal injection with a mix containing Ketamine (75mg/kg) and Medetomidine (1mg/kg). Mouse lungs were intracardially perfused with 20 ml ice cold PBS, then lungs were removed and intratracheally inoculated with 2 ml of 5 U/ml dispase (Corning). After 10 min incubation at 37°C, lungs were cut into small pieces with surgical blades (Braun) and transferred to GentleMacs M Tubes (Miltenyi) containing 7 ml HBSS, 1% FBS, 20 U/ml DNase I (Promega) and 70 U/ml collagenase I (Gibco). Samples were homogenised in a GentleMacs Dissociator (Miltenyi) using the "mouse lung 01" program, incubated for 30 min at 37 °C under shaking and homogenised again with the "mouse lung 02" GentleMacs Dissociator program. The homogenised mixture was subsequently filtered through 70 nm and 40mm cell strainers (BD) and centrifuged at 300 x g for 5 min. The cell pellet was resuspended in 2 ml Quiagen lysis buffer for 4 min at RT for red blood cell lysis. Cells were then resuspended in 13 ml DMEM + 10%FBS. Cell were counted and viability was assessed using trypan blue (Sigma) in an automated cell counter Countess (Molecular Probes), and resuspended at 107 cells/ml in HBSS + 2% FBS containing 10 mM HEPES buffer (Gibco). Lung samples were stained with antiCD31 APC (dilution 1/400, BD, #551260) and antiCD45 APC (dilution 1/200, BD, #559864), and antiCD326 (EpCAM) AF488 (dilution 1/200, BD, #118210) rat monoclonal conjugated antibodies. DAPI (Sigma) was added for live/dead discrimination to a final concentration of 20 ng/ml. All samples were acquired and sorted using an InFlux (BD) equipped with 640nm, 561nm, 488nm and 355nm lines. OneComp Beads (eBioscience) were used as compensation controls to assess and correct spectral overlapping. Population gating was performed using fluorescence minus one (FMO) controls (Fig. 7f, g). Pulse processing was used to exclude cell aggregates, and at least 50,000 events were collected from the live CD45-CD31- gate for analysis.

### RNAseq

Total RNA from flow-sorted lung cells was extracted with an RNAeasy mini kit (Quiagen). Approximately 2 ng of total RNA samples (range 0.4-5 ng) were processed with the SMART-Seq v4 Ultra Low Input RNA Kit (Clontech) by following manufacturer instructions. Resulting cDNA was sheared on a S220 Focused-ultrasonicator (Covaris) and subsequently processed with the "NEBNext Ultra II DNA Library Prep Kit for Illumina" (NEB #E7645). Briefly, oligo(dT)-primed reverse transcription was performed in the presence of a template switching oligonucleotide, double stranded cDNA was produced by 14 cycles of PCR and submitted to acoustic shearing. Fragments were processed through subsequent enzymatic treatments of end-repair, dA-tailing, and ligation to Illumina adapters. Adapter-ligated libraries were completed by limited-cycle PCR (8 cycles). The resulting purified cDNA libraries were applied to an Illumina flow cell for cluster generation and sequenced on an Illumina HiSeq2500 by following manufacturer's protocols. Image analysis, per-cycle base calling, and quality score assignment was performed with Illumina Real Time Analysis software. Conversion of Illumina BCL files to bam format was performed with the Illumina2bam tool (Wellcome Trust Sanger Institute - New Pipeline Group). Sequencing quality was analyzed with FastQC; reads were aligned to the mouse genome (GRCm38/mm10) using TopHat-2.0.10 (Trapnell *et al.,* 2012), Bowtie 1.0.0 (Langmead *et al.,* 2009) and Samtools 0.1.19.0 (Li *et al.,* 2009); and transcripts assembly, abundances estimation and differential expression were calculated with DESeq2 (Love, Huber and Anders, 2014). The estimated significance level (P value) was corrected to account for multiple hypotheses testing using Benjamini and Hochberg False Discovery Rate (FDR) adjustment. Genes with FDR less than or equal to 0.05 were considered differentially expressed genes. GSEA of differentially expressed genes was performed using the GSEA software v2.0.6, obtained from the Broad Institute and following the developer's protocol. The previously obtained differentially expressed genes were ranked according to their t-statistic. This ranked file was used as input for the enrichment analysis. All basic and advanced fields were set to default and only gene sets significantly enriched at a FDR q-values < 0.25 were considered. The primary RNA-seq data has been deposited in the GEO repository under accession number GSE97685.

### Analysis of mRNA levels

For lung samples, total RNA from flow-sorted lung cells was extracted with an RNAeasy mini kit (Qiagen, #74106). For heart samples, total RNA was isolated by acid guanidinium thiocyanate-phenol-chloroform extraction. Up to 4 µg of total RNA was reverse transcribed into cDNA using iScriptTM Advanced cDNA Synthesis Kit for RT- qPCR (BioRad #172-5038). For qRT-PCR, each cDNA sample was assayed in triplicate reactions in opticalgrade, 386-well plates (Applied Biosystems). Each reaction contained 6 µl of SybrGreen (2x) (Applied Biosystems), 2 µl of cDNA template, 3.4 µl of DNAse-free water and 0.6 µl of primers (10 µM f.c.). All PCR runs were performed on a QuantStudioTM 6 Flex Real-Time PCR System using QuantStudioTM 6 and 7 Flex Real-Time PCR software v1.0 (Applied Biosystems). Reaction conditions were as follows: 10 min at 95°C followed by 40 cycles of 15 sec at 95°C, 1 min at 60°C and 1 min at 62°C. The housekeeping genes Actb and/or Gapdh were used for input normalization, as indicated.

List of primers used for mRNA expression analyses:
*Actb* Forward: 5'-GGCACCACACCTTCTACAATG-3' (SEQ ID NO:i)
*Actb* Reverse: 5'-GTGGTGGTGAAGCTGTAGCC-3' (SEQ ID NO:2)
*Gapdh* Forward: 5'-TTCACCACCATGGAGAAGGC-3' (SEQ ID NO:3)
*Gapdh* Reverse: 5'-CCCTTTTGGCTCCACCCT-3' (SEQ ID NO:4)
*Il6* Forward: 5'-GTTCTCTGGGAAATCGTGGA-3' (SEQ ID NO:5)
*Il6* Reverse: 5'-GGTACTCCAGAAGACCAGAGGA-3' (SEQ ID NO:6)
*Myh7* Forward: 5'-TGCAGCAGTTCTTCAACCAC-3' (SEQ ID NO:7)
*Myh7* Reverse: 5'-TCGAGGCTTCTGGAAGTTGT-3' (SEQ ID NO:8)
*Nppa* Forward: 5'-ATCTGCCCTCTTGAAAAGCA-3' (SEQ ID NO:9)
*Nppa* Reverse: 5'-ACACACCACAAGGGCTTAGG-3' (SEQ ID NO:10)

### SA-βgal activity assay

SA-βgal staining was performed in whole-mount tissues (either lungs or tumour xenografts) or in tissue cryosections (either lungs or tumour xenografts) preserved in OCT using the Senescence β-Galactosidase Staining Kit (Cell Signalling). In cells, SAβG staining was performed following the manufacturer's instructions. Briefly, whole-mount tissues or tissue cryosections were fixed at room temperature, for 45 min or 2 min, respectively, with a solution containing 2% formaldehyde and 0.2% glutaraldehyde in PBS, washed three times with PBS, and incubated overnight at 37°C with the Staining Solution containing X-gal in N-N-dimethylformamide (pH 6.0). In the case of whole-mount staining, embryos were subsequently dehydrated with two consecutive steps in 50% and 70% EtOH and embedded in paraffin for serial sectioning. Sections were counterstained with nuclear fast red (NFR) or were processed for immunohistochemistry.

### Lung in vivo imaging by CT

The acquisition was made on a CT Locus high-resolution computed tomography system (GE Healthcare) specifically designed for small laboratory animals and with an isotropic resolution of 45 µm. Mice were anesthetised with a 4% rate of isoflurane (IsoVet Braun) during the induction and 2% during the maintenance period (scanning time). Micro-CT image acquisition consisted of 400 projections collected in one full rotation of the gantry during approximately 10 min in a single bed focused on the legs, with a 450-mA/80-kV X-ray tube. The resulting raw data were reconstructed to a final image volume of 875 × 875 × 465 slices at (93 µm) 3 voxel dimensions. 2D and 3D images were obtained and analysed by using the software program Micro View 2.2 (GE Healthcare) to calculate the total volume area of the lungs and the fibrotic volume. Image analysis quantification was performed by an independent and well-trained researcher in a blind manner. All procedures were carried out according to the European Normative of Welfare and Good Practice (2010/63/UE).

### In vivo and ex vivo imaging by IVIS

An IVIS Spectrum Imaging System (Perkin Elmer Inc) was used for in vivo and ex vivo fluorescence imaging. For *in vivo* imaging, mice were anesthetized with a 4% rate of isoflurane (IsoVet Braun) during the induction, and 2% during the maintenance period (scanning time). For *ex vivo* imaging, mice were sacrificed by CO2 exposure in a euthanasia chamber, and organs and tumor xenografts were analyzed immediately after harvesting.

Rhodamine B was detected using excitation wavelength of 535 nm and emission wavelength of 580 nm. Indocyanine green was detected using excitation passband of 710-760 nm and emission passband of 810-875 nm. Doxorubicin was detected using excitation wavelength of 430 nm and emission wavelength of 600 nm. Fluorescence imaging quantification was performed by Living Image 3.2 software (Perkin Elmer Inc). A region of interest area (ROI) was drawn over the fluorescent signal either in lung or xenograft tumour samples in order to quantify the release of the fluorophores. Fluorescence activity is measured in photons per second per square centimetre per steradian (p/s⁻¹ cm⁻² sr⁻¹).

### Blood analysis

Blood samples were obtained by sub-mandibular blood extraction and collected in EDTA-coated tubes (Aquisel). For hepatic and renal function assessment, blood samples were centrifuged at 2,000x g for 10 min, and the serum (supernatant) was removed. Serum samples were added at a volume of 50ul into VetScan Mammalian Liver Profile reagent rotors PN:500-1040 (Abaxis). Hepatic and renal profiles were assessed by analysis of the rotors with a VetScan Chemistry Analyzer (Abaxis).

### Plethysmography

Mice were anesthetised by intraperitoneal injection with a mix containing Ketamine (75mg/kg) and Medetomidine (1mg/kg). Animals were placed on a Tilting WorkStand for rodents (EMC Hallowell) and intubated intratracheally with a 24GA catheter (BD Biosciences). Then animals were placed in a plethysmograph adapted for anesthetized rodents (emka TECHNOLOGIES). A MiniVent Model 845 (Harvard Apparatus) connected to the plethysmograph was used to ventilate the mice at a frequency of 150 beats/min and 10 mL/kg of tidal volume. Transpulmonary pressure was measured with a pressure transducer connected to the cannula. Airflow and tidal volume were determined using a flow transducer fixed to the plethysmograph chamber. Lung resistance (LR) and compliance dynamics (Cdyn) were calculated and analysed with iox2 software (emka TECHNOLOGIES).

### Silica biodistribution assays

Mice were sacrificed 6h post GalNP(rho) injection and several selected organs were extracted (lungs, liver, spleen, bladder, kidneys, heart, gut and tail) for signal intensity (Si) detection.

The extracted organs were first weighed and then digested with 1 mL of tetramethylammonium hydroxide solution 25% in H₂O (TMHA, Sigma). Digestion was carried out in closed polytetrafluoroethylene (PTFE) vessels for 2 h at 80°C, using a digestion unit Bloc digest 20 (Selecta). After cooling, digested samples were diluted to 10 ml in polypropylene Erlenmeyer flasks (in order to avoid Si contamination) and kept in polystyrene tubes. For the analysis, 0.5 ml of the digested samples were diluted to 10 mL with a solution of nitric acid 2% and hydrochloric acid 1%. Standard solutions were prepared from silicon standard for Inductively Coupled Plasma Mass Spectroscopy (ICP-MS) (1000 mg/L Si in NaOH, Sigma) and were digested and treated exactly the same way as the samples. Samples were analysed with an ICP-MS System Agilent 7900. Results were calculated using calibration with digested standard solutions. Data are expressed as µg Si/g sample.

### Statistical methods

Statistical significance was assessed using Student's t-test (two-tailed, unpaired). Data were represented as the mean of the indicated experimental replicates. Error bars represent the standard deviation (SD) or the standard error of the mean (SEM), as indicated in the figure legends. *p < 0.05; **p < 0.01; ***p < 0.001.

### Results

### Example 1: GalNP are preferentially activated in senescent cells

The inventors previously reported nanoparticles that release their cargoes preferentially within *in vitro* cultured senescent cells (Agostini *et al.,* 2012). Their strategy was based upon the high lysosomal *β*-galactosidase activity generally associated with senescent cells, also known as SA-*β*gal (senescence-associated *β*-galactosidase) (Dimri *et al.,* 1995). In particular, the inventors previously reported GOS-coated nanoparticles or GosNP (Figure 7b), where the coating of the nanoparticles consists in a heterogeneous mixture containing at least 60% of galacto-oligosaccharides; 33% disaccharides, 39% trisaccharides, 18% tetrasaccharides, 7% pentasaccharides and 3% of higher saccharides, provided by Zeus Química (Spain) (Figures 2, 3). One technical novelty of the current invention was to encapsulate the internal cargo of the nanoparticles with a homogeneous coating of 6-mer galacto-oligosaccharides (see the specific length of the homogenous 6-mer galacto-oligosaccharides in Figures 2, 3). In particular, in the Figure 7b the improvement release profile was shown. For this, inventors used purified galacto-oligosaccharides composed by 6-mers (Figures 2, 3,6) provided by Carbosynth (UK), and known as GALACTAN, and the resulting nanoparticules are referred to as GalNP (Figure 7b). Another technical novelty is that the inventors modified the method of linking the sugar coating to the silica scaffold. In particular, the GosNP synthesis involved the following sequence of reactions: first, linking the sugars to an adaptor; and, second, linking the adaptor-sugar to the silica scaffold (Agostini *et al.,* 2012). In the current invention, GalNP synthesis involved a different sequence of reactions: first, linking the adaptor to the silica scaffold; and, second, linking the sugar to the adaptor-scaffold, see Figure 7a.

Surprising and advantageously, the two above-mentioned technical novelties provided a more controlled release of the cargo concerned. Inventors generated nanoparticles loaded with rhodamine B [GalNP(rho)] and doxorubicin [GalNP(dox)], and enzymatic digestion of these three types of nanoparticles with a bacterial *β*-galactosidase resulted in efficient release of their respective cargos (Figure 7b). The fluorescence of cargos like rhodamine B and doxorubicin is mostly quenched when these fluorophores are encapsulated within the nanoparticles; and fluorescence is maximal when the fluorescent cargos are released. It is important to note that in the absence of *β*-galactosidase, the amount of cargo spontaneously released is lower in the case of GalNP(rho) compared to GosNP(rho) (Figure 7b).

In general, 100 mg of drug are encapsulated per 1 g of beads and, upon digestion in vitro, ∼30 mg of drug are released per g of beads (Tables 2, 3).

In light of the above, the inventors were able to conclude that their nanoparticle strategy may be used as a versatile and efficient platform to deliver small compounds into damaged and/or senescent cells.

### Example 2: GalNP(rho) release in pulmonary fibrosis

Cellular senescence is abundant in pulmonary fibrosis, both in humans and in mice, and actively contributes to the pathological manifestations of this disease (Aoshiba, Tsuji and Nagai, 2003; Aoshiba *et al.,* 2013; Hecker *et al.,* 2014; Pan *et al.,* 2017; Schafer *et al.,* 2017).

The inventors wondered whether this senescence delivery system would also work in this model of in vivo senescence. Intratracheal instillation of bleomycin in mice produced full-blown lung fibrosis in a period of two weeks, accompanied by focal areas of SAbGal activity and strong collagen deposition (Fig. 8a). After 2 weeks of post-bleomycin administration, mice were intravenously injected with GalNP(rho) and 6 hours later fluorescence was measured in the lungs. Importantly, rhodamine release occurred preferentially in fibrotic lungs compared to healthy lungs (Fig. 8b). The inventors wondered if the GalNP beads would retain their activity when administered intratracheally rather than intravenously. Indeed, intratracheal administration of the beads was also efficient in targeting fibrotic lungs (Fig. 9a). Upon tail vein injection, silica beads accumulate initially in the lungs (during the first hours post-injection) and then in the spleen and liver (24h post-injection) (Yu *et al.,* 2012).

The inventors confirmed that this was the case for the GalNP(rho) beads, as deduced by the levels of silicon in the lungs of control and bleomycin-treated mice at 6h post-injection (Fig. 9b). Also, confocal microscopy indicated that Rho+ cells were more abundant in fibrotic lung lesions compared to non-fibrotic lungs (Fig. 10a). Therefore, the silica beads reach equally well both healthy and fibrotic lungs (Fig. 9b), however, the release of the fluorophore preferentially occurs within fibrotic lungs (Fig. 8b and Fig. 10a). Next, the inventors set to characterize in further detail the cells targeted by GalNP(rho) in fibrotic lungs using flow cytometry. After excluding endothelial (CD31+) and hematopoietic (CD45+) cells (Fig. 9c, d), the inventors quantified the relative number of Rho+ cells in double negative CD45-CD31-cells, which are mostly comprised by lung epithelial cells and fibroblasts. Importantly, bleomycin-treated lungs showed higher levels of Rho+CD45- CD31- cells than control lungs (Fig. 10b). Further analysis using the epithelial marker EpCAM suggested that the large majority of Rho+CD45-CD31- cells corresponded to fibroblasts (EpCAM-) (Fig. 10b). To directly test whether Rho+CD45-CD31- cells are indeed senescent, CD45-CD31- cells from bleomycin-treated lungs were sorted into Rho+ and Rho subpopulations and subjected to RNAseq. Gene set enrichment analyses (GSEA) using published signatures of senescence (Lasry and Ben-Neriah, 2015) indicated that Rho+CD45-CD31- cells present a significant upregulation of senescence signatures (Fig. 10c). IL6 is a relevant cytokine produced by senescent cells (Kuilman *et al.,* 2008; Mosteiro *et al.,* 2016), and the inventors confirmed by qRT-PCR the upregulation of Il6 in Rho+CD45-CD31- cells from bleomycin-treated lungs (Fig. 10d).

These results demonstrate that GalNP nanoparticles release their cargoes preferentially within senescent cells present in a damaged tissue. In addition, they also show that GalNP can be used as an *in vivo* tool to detect and isolate senescent cells from tissues.

### Example 3: Therapeutic effect of cytotoxic GalNP on pulmonary fibrosis

Based on the above data, the inventors wondered whether gal-encapsulated doxorubicin could be therapeutic on bleomycin-induced pulmonary fibrosis, a disease that is caused, at least in part, by the accumulation of senescent cells (Pan *et al.,* 2017; Schafer *et al.,* 2017). Bleomycin was administered intratracheally and, 10 days later, mice were treated with free doxorubicin or with GalNP(dox) (Fig. 11a). The inventors confirmed that GalNP(dox) efficiently released doxorubicin in lungs from bleomycin treated mice, but not in healthy lungs, as determined by total fluorescence (Fig. 12a). At 10 days post-bleomycin, lung function was assessed by pletysmography (Milton *et al.,* 2012). As expected, all the mice treated with bleomycin presented a significant increase in the L_{R}/C_{dyn} ratio (L_{R}: lung resistance; C_{dyn}: compliance dynamics), which is indicative of pulmonary fibrosis. Bleomycin-treated mice were subsequently treated with daily doses of free or gal-encapsulated doxorubicin (1 mg/kg) for ∼2 weeks. Importantly, at the end of the treatment, mice treated with GalNP(dox), but not with free doxorubicin, presented L_{R/}C_{dyn} values similar to those of healthy controls (Fig. 11b). These data were confirmed by computerised tomography (CT), which indicated a significant reduction in the lung volume affected by inflammation and fibrosis in GalNP(dox)-treated mice (Fig. 11c). Histological analysis of the bleomycin-treated lungs showed characteristic features of lung fibrosis, namely, focal areas of high cellularity with interstitial thick collagen deposits (stained with sirius red) (Fig. 11d).

Similar lesions were observed in mice that had been subsequently treated with free doxorubicin (Fig. 11d). In contrast, mice that had been subsequently treated with gal-encapsulated doxorubicin for ∼2 weeks presented a significant collagen reduction in the damaged areas of bleomycin-treated lungs (Fig. 11d). No evidence of hepatic or renal damage was observed in any of the groups, as evaluated by serum markers and histology (Fig. 12b, c). Collectively, these results indicate that the encapsulation of a cytotoxic drug, such as doxorubicin, into GalNP generates a new therapeutic entity that targets senescent lesions and has therapeutic activity against pulmonary fibrosis.

### Conclusion

As a result of the present research, the inventors have shown that their nanoparticles are surprisingly able to release their payloads within damaged and/or senescent cells in a controlled manner. Administration of nanoparticles carrying a fluorophore has been found by the inventors to result in the fluorescent labelling of senescent lesions in mice. In addition, the nanoparticles have been shown by the inventors to target senescent cells in bleomycin-induced pulmonary fibrosis, reduce collagen deposition, and ameliorate pulmonary function. Accordingly, the present invention opens up a raft of new diagnostic and therapeutic methods that can be used to diagnose and treat the multiple disorders known to be associated with damaged and/or senescent cells

### References

Agostini, A., Mondragõn, L., Bernardos, A., Martínez-Máñez, R., Dolores Marcos, M., Sancenõn, F., Soto, J., Costero, A., Manguan-García, C., Perona, R., Moreno-Torres, M., Aparicio-Sanchis, R. and Murguía, J. R. (2012) 'Targeted cargo delivery in senescent cells using capped mesoporous silica nanoparticles', Angewandte Chemie - International Edition, 51(42), pp. 10556-10560. doi: 10.1002/anie.201204663.
Aoshiba, K., Tsuji, T., Kameyama, S., Itoh, M., Semba, S., Yamaguchi, K. and Nakamura, H. (2013) 'Senescence-associated secretory phenotype in a mouse model of bleomycin-induced lung injury', Experimental and Toxicologic Pathology, 65(7-8), pp. 1053-1062. doi: 10.1016/j.etp.2013.04.001.
Aoshiba, K., Tsuji, T. and Nagai, A. (2003) 'Bleomycin induces cellular senescence in alveolar epithelial cells', European Respiratory Journal, 22(3), pp. 436-443. doi: 10.1183/09031936.03.00011903.
Baker, D. J., Childs, B. G., Durik, M., Wijers, M. E., Sieben, C. J., Zhong, J., Saltness, R. A., Jeganathan, K. B., Verzosa, G. C., Pezeshki, A., Khazaie, K., Miller, J. D. and van Deursen, J. M. (2016) 'Naturally occurring p16(Ink4a)-positive cells shorten healthy lifespan.', Nature, 530(7589), pp. 184-9. doi: 10.1038/nature16932.
Chang, J., Wang, Y., Shao, L., Laberge, R. M., Demaria, M., Campisi, J., Janakiraman, K., Sharpless, N. E., Ding, S., Feng, W., Luo, Y., Wang, X., Aykin-Burns, N., Krager, K., Ponnappan, U., Hauer-Jensen, M., Meng, A. and Zhou, D. (2016) 'Clearance of senescent cells by ABT263 rejuvenates aged hematopoietic stem cells in mice', Nat Med, 22(1), pp. 78-83. doi: 10.1038/nm.4010.
Childs, B. G., Baker, D. J., Wijshake, T., Conover, C. A., Campisi, J. and van Deursen, J. M. (2016) 'Senescent intimal foam cells are deleterious at all stages of atherosclerosis.', Science (New York, N.Y.), 354(6311), pp. 472-477. doi: 10.1126/science.aaf6659.
Cho, J.-H., Saini, D. K., Karunarathne, W. K. A., Kalyanaraman, V. and Gautam, N. (2011) 'Alteration of Golgi structure in senescent cells and its regulation by a G protein γ subunit.', Cellular signalling, 23(5), pp. 785-93. doi: 10.1016/j.cellsig.2011.01.001.
Demaria, M., O'Leary, M. N., Chang, J., Shao, L., Liu, S., Alimirah, F., Koenig, K., Le, C., Mitin, N., Deal, A. M., Alston, S., Academia, E. C., Kilmarx, S., Valdovinos, A., Wang, B., de Bruin, A., Kennedy, B. K., Melov, S., Zhou, D., Sharpless, N. E., Muss, H. and Campisi, J. (2016) 'Cellular Senescence Promotes Adverse Effects of Chemotherapy and Cancer Relapse', Cancer Discovery*.*
Dimri, G. P., Lee, X., Basile, G., Acosta, M., Scott, G., Roskelley, C., Medrano, E. E., Linskens, M., Rubelj, I. and Pereira-Smith, O. (1995) 'A biomarker that identifies senescent human cells in culture and in aging skin in vivo.', Proceedings of the National Academy of Sciences of the United States of America, 92(20), pp. 9363-7. doi: 10.1073/pnas.92.20.9363.
Feng, C., Liu, H., Yang, M., Zhang, Y., Huang, B. and Zhou, Y. (2016) 'Disc cell senescence in intervertebral disc degeneration: Causes and molecular pathways', Cell Cycle, pp. 1674-1684. doi: 10.1080/15384101.2016.1152433.
Hecker, L., Logsdon, N. J., Kurundkar, D., Kurundkar, A., Bernard, K., Hock, T., Meldrum, E., Sanders, Y. Y. and Thannickal, V. J. (2014) 'Reversal of Persistent Fibrosis in Aging by Targeting Nox4-Nrf2 Redox Imbalance', Science Translational Medicine, 6(231), p. 231ra47-231ra47. doi: 10.1126/scitranslmed.3008182.
Herranz, N., Gallage, S., Mellone, M., Wuestefeld, T., Klotz, S., Hanley, C. J., Raguz, S., Acosta, J. C., Innes, A. J., Banito, A., Georgilis, A., Montoya, A., Wolter, K., Dharmalingam, G., Faull, P., Carroll, T., Martinez-Barbera, J. P., Cutillas, P., Reisinger, F., Heikenwalder, M., Miller, R. A., Withers, D., Zender, L., Thomas, G. J. and Gil, J. (2015) 'mTOR regulates MAPKAPK2 translation to control the senescence-associated secretory phenotype', Nature Cell Biology, 17(9), pp. 1205-1217. doi: 10.1038/ncb3225.
Ivanov, A., Pawlikowski, J., Manoharan, I., van Tuyn, J., Nelson, D. M., Rai, T. S., Shah, P. P., Hewitt, G., Korolchuk, V. I., Passos, J. F., Wu, H., Berger, S. L. and Adams, P. D. (2013) 'Lysosome-mediated processing of chromatin in senescence.', The Journal of cell biology. Rockefeller University Press, 202(1), pp. 129-43. doi: 10.1083/jcb.201212110.
Knas, M., Zalewska, A., Kr 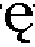 towski, R., Niczyporuk, M., Waszkiewicz, N., Cechowska-Pasko, M., Waszkiel, D. and Zwierz, K. (2012) 'The profile of lysosomal exoglycosidases in replicative and stress-induced senescence in early passage human fibroblasts', *Folia Histochemica et Cytobiologica,* 50(2), pp. 220-227. doi: 10.5603/FHC.2012.0031.
Kuilman, T., Michaloglou, C., Vredeveld, L. C. W., Douma, S., van Doom, R., Desmet, C. J., Aarden, L. A., Mooi, W. J. and Peeper, D. S. (2008) 'Oncogene-induced senescence relayed by an interleukin-dependent inflammatory network.', Cell, 133(6), pp. 1019-31. doi: 10.1016/j.cell.2008.03.039.
Laberge, R.-M., Sun, Y., Orjalo, A. V, Patil, C. K., Freund, A., Zhou, L., Curran, S. C., Davalos, A. R., Wilson-Edell, K. A., Liu, S., Limbad, C., Demaria, M., Li, P., Hubbard, G. B., Ikeno, Y., Javors, M., Desprez, P.-Y., Benz, C. C., Kapahi, P., Nelson, P. S. and Campisi, J. (2015) 'MTOR regulates the pro-tumorigenic senescence-associated secretory phenotype by promoting ILiA translation.', Nature cell biology, 17(8), pp. 1049-61. doi: 10.1038/ncb3195.
Langmead, B., Trapnell, C., Pop, M. and Salzberg, S. L. (2009) 'Ultrafast and memory-efficient alignment of short DNA sequences to the human genome', Genome Biology. BioMed Central, 10(3), p. R25. doi: 10.1186/gb-2009-10-3-r25.
Lasry, A. and Ben-Neriah, Y. (2015) 'Senescence-associated inflammatory responses: Aging and cancer perspectives', Trends in Immunology, pp. 217-228. doi: 10.1016/j.it.2015.02.009.
Li, H., Handsaker, B., Wysoker, A., Fennell, T., Ruan, J., Homer, N., Marth, G., Abecasis, G., Durbin, R. and 1000 Genome Project Data Processing Subgroup (2009) 'The Sequence Alignment/Map format and SAMtools', Bioinformatics, 25(16), pp. 2078-2079. doi: 10.1093/bioinformatics/btp352.
Love, M. I., Huber, W. and Anders, S. (2014) 'Moderated estimation of fold change and dispersion for RNA-seq data with DESeq2', Genome Biology. BioMed Central, 15(12), p. 550. doi: 10.1186/s13059-014-0550-8.
Lujambio, A. (2016) 'To clear, or not to clear (senescent cells)? That is the question', BioEssays, 38, pp. S56-S64. doi: 10.1002/bies.201670910.
Milton, P. L., Dickinson, H., Jenkin, G. and Lim, R. (2012) 'Assessment of Respiratory Physiology of C57BL/6 Mice following Bleomycin Administration Using Barometric Plethysmography', Respiration, 83(3), pp. 253-266. doi: 10.1159/000330586.
Mosteiro, L., Pantoja, C., Alcazar, N., Marión, R. M., Chondronasiou, D., Rovira, M., Fernandez-Marcos, P. J., Muñoz-Martin, M., Blanco-Aparicio, C., Pastor, J., Gómez-Lopez, G., De Martino, A., Blasco, M. A., Abad, M. and Serrano, M. (2016) 'Tissue damage and senescence provide critical signals for cellular reprogramming in vivo.', Science (New York, N.Y.), 354(6315). doi: 10.1126/science.aaf4445.
Muñoz-Espín, D., Cañamero, M., Maraver, A., Gómez-López, G., Contreras, J., Murillo-Cuesta, S., Rodriguez-Baeza, A., Varela-Nieto, I., Ruberte, J., Collado, M. and Serrano, M. (2013) 'Programmed cell senescence during mammalian embryonic development.', Cell, 155(5), pp. 1104-18. doi: 10.1016/j.cell.2013.10.019.
Muñoz-Espín, D. and Serrano, M. (2014) 'Cellular senescence: From physiology to pathology', Nature Reviews Molecular Cell Biology, pp. 482-496. doi: 10.1038/nrm3823.
Narita, M., Young, A. R. J., Arakawa, S., Samarajiwa, S. A., Nakashima, T., Yoshida, S., Hong, S., Berry, L. S., Reichelt, S., Ferreira, M., Tavare, S., Inoki, K., Shimizu, S. and Narita, M. (2011) 'Spatial Coupling of mTOR and Autophagy Augments Secretory Phenotypes', Science, 332(6032), pp. 966-970. doi: 10.1126/science.1205407.
Pan, J., Li, D., Xu, Y., Zhang, J., Wang, Y., Chen, M., Lin, S., Huang, L., Chung, E. J., Citrin, D. E., Wang, Y., Hauer-Jensen, M., Zhou, D. and Meng, A. (2017) 'Inhibition of Bcl-2/xl With ABT-263 Selectively Kills Senescent Type II Pneumocytes and Reverses Persistent Pulmonary Fibrosis Induced by Ionizing Radiation in Mice', International Journal of Radiation Oncology Biology Physics, 99(2), pp. 353-361. doi: 10.1016/j.ijrobp.2017.02.216.
Roos, C. M., Zhang, B., Palmer, A. K., Ogrodnik, M. B., Pirtskhalava, T., Thalji, N. M., Hagler, M., Jurk, D., Smith, L. A., Casaclang-Verzosa, G., Zhu, Y., Schafer, M. J., Tchkonia, T., Kirkland, J. L. and Miller, J. D. (2016) 'Chronic senolytic treatment alleviates established vasomotor dysfunction in aged or atherosclerotic mice.', Aging cell, 15(5), pp. 973-7. doi: 10.nn/ acel.12458.
Schafer, M. J., White, T. A., Iijima, K., Haak, A. J., Ligresti, G., Atkinson, E. J., Oberg, A. L., Birch, J., Salmonowicz, H., Zhu, Y., Mazula, D. L., Brooks, R. W., Fuhrmann-Stroissnigg, H., Pirtskhalava, T., Prakash, Y. S., Tchkonia, T., Robbins, P. D., Aubry, M. C., Passos, J. F., Kirkland, J. L., Tschumperlin, D. J., Kita, H. and LeBrasseur, N. K. (2017) 'Cellular senescence mediates fibrotic pulmonary disease', Nature Communications, 8, p. 14532. doi: 10.1038/ncomms14532.
Storer, M., Mas, A., Robert-Moreno, A., Pecoraro, M., Ortells, M. C., Di Giacomo, V., Yosef, R., Pilpel, N., Krizhanovsky, V., Sharpe, J. and Keyes, W. M. (2013) 'Senescence is a developmental mechanism that contributes to embryonic growth and patterning.', Cell, 155(5), pp. 1119-30. doi: 10.1016/j.cell.2013.10.041.
Tai, H., Wang, Z., Gong, H., Han, X., Zhou, J., Wang, X., Wei, X., Ding, Y., Huang, N., Qin, J., Zhang, J., Wang, S., Gao, F., Chrzanowska-Lightowlers, Z. M., Xiang, R. and Xiao, H. (2017) 'Autophagy impairment with lysosomal and mitochondrial dysfunction is an important characteristic of oxidative stress-induced senescence', Autophagy, 13(1), pp. 99-113. doi: 10.1080/15548627.2016.1247143.
Trapnell, C., Roberts, A., Goff, L., Pertea, G., Kim, D., Kelley, D. R., Pimentel, H., Salzberg, S. L., Rinn, J. L. and Pachter, L. (2012) 'Differential gene and transcript expression analysis of RNA-seq experiments with TopHat and Cufflinks', Nature Protocols, 7(3), pp. 562-578. doi: 10.1038/nprot.2012.016.
Udono, M., Fujii, K., Harada, G., Tsuzuki, Y., Kadooka, K., Zhang, P., Fujii, H., Amano, M., Nishimura, S. I., Tashiro, K., Kuhara, S. and Katakura, Y. (2015) 'Impaired ATP6VoA2 expression contributes to Golgi dispersion and glycosylation changes in senescent cells', Scientific Reports, 5. doi: 10.1038/srep17342.
Yosef, R., Pilpel, N., Tokarsky-Amiel, R., Biran, A., Ovadya, Y., Cohen, S., Vadai, E., Dassa, L., Shahar, E., Condiotti, R., Ben-Porath, I. and Krizhanovsky, V. (2016) Directed elimination of senescent cells by inhibition of BCL-W and BCL-XL.', Nature communications, 7, p. 11190. doi: 10.1038/ncomms11190.
Yu, T., Hubbard, D., Ray, A. and Ghandehari, H. (2012) 'In vivo biodistribution and pharmacokinetics of silica nanoparticles as a function of geometry, porosity and surface characteristics', in Journal of Controlled Release, pp. 46-54. doi: 10.1016/j.jconrel.2012.05.046.
Zhu, Y., Tchkonia, T., Fuhrmann-Stroissnigg, H., Dai, H. M., Ling, Y. Y., Stout, M. B., Pirtskhalava, T., Giorgadze, N., Johnson, K. O., Giles, C. B., Wren, J. D., Niedernhofer, L. J., Robbins, P. D. and Kirkland, J. L. (2016) 'Identification of a novel senolytic agent, navitoclax, targeting the Bcl-2 family of anti-apoptotic factors.', Aging cell, 15(3), pp. 428-35. doi: 10.1111/acel.12445.
Zhu, Y., Tchkonia, T., Pirtskhalava, T., Gower, A. C., Ding, H., Giorgadze, N., Palmer, A. K., Ikeno, Y., Hubbard, G. B., Lenburg, M., O'Hara, S. P., LaRusso, N. F., Miller, J. D., Roos, C. M., Verzosa, G. C., LeBrasseur, N. K., Wren, J. D., Farr, J. N., Khosla, S., Stout, M. B., McGowan, S. J., Fuhrmann-Stroissnigg, H., Gurkar, A. U., Zhao, J., Colangelo, D., Dorronsoro, A., Ling, Y. Y., Barghouthy, A. S., Navarro, D. C., Sano, T., Robbins, P. D., Niedernhofer, L. J. and Kirkland, J. L. (2015) 'The Achilles' heel of senescent cells: from transcriptome to senolytic drugs.', Aging cell, 14(4), pp. 644-58. doi: 10.1111/acel.12344.

## Claims

1. A nanoparticle arranged, in use, to encapsulate and deliver a payload molecule to a damaged and/or senescent cell, the nanoparticle comprising at least one cavity or reservoir in which a payload is contained, the at least one cavity being gated by one or more oligosaccharides, wherein each cavity of said nanoparticle is gated by the same at least one or more oligosaccharides.

2. A nanoparticle according to claim 1, wherein the nanoparticle comprises 1, 10, 20, 30, 40, 50, 60, 70, 80, 90, 100, 250, 500, 750, 1000, 1200, 1300, 1400, 1500, 1600, 1700, 2000, 2200, 2400 or more external cavity/cavities.

3. A nanoparticle according to claim 2, wherein the payload molecule is a drug, cytotoxic compound or senolytic compound.

4. A nanoparticle according to any of the preceding claims, wherein at least 30%, 40%, 50%, 60%, 70%, 80%, 90%, 95% or 100% of the unit/s of monosaccharide may be galactose and/or fucose.

5. A nanoparticle according to any of the preceding claims, comprising between 1 and 10000, or between 2000 and 8000, or between 2000 and 6000, or between 3000 and 5000, or between 3500 and 5500, or between 4000 and 5000 oligosaccharides.

6. A nanoparticle according to any of the preceding claims, wherein the one or more oligosaccharide(s) are the same or different with respect to length and composition, provided the nanoparticle comprises a homogeneous distribution of them on its surface.

7. A nanoparticle according to any of the preceding claims, wherein the at least one cavity is gated by at least 1, 2, 3, 4, 6, 8, 10 or more oligosaccharides.

8. A nanoparticle according to any of the preceding claims, wherein at least 55%, 65%, 75%, 85%, 95% or 100% of the nanoparticle surface is coated with said oligosaccharides.

9. A nanoparticle according to any of the preceding claims, wherein a homogeneous coating of 6-mer galacto-oligosaccharides is present at each cavity on the surface of the nanoparticles.

10. A nanoparticle according to any of the preceding claims, wherein the nanoparticle is produced by:-
(i) creating a nanoparticle with at least one cavity;
(ii) loading the at least one cavity with a payload; and
(iii) coating the loaded nanoparticle with oligosaccharide, so that the cavity is gated with one or more oligosaccharide(s).

11. A cell comprising a nanoparticle according to any one of claims 1 to 10, which is a bacterial, yeast or animal cell.

12. A nanoparticle according to any one of claims 1 to 10, or a cell according to claim 11, for use in eliminating damaged and/or senescent cells.

13. A nanoparticle according to any one of claims 1 to 10, or a cell according to any one of claim 11, for use in the treatment, amelioration, or prevention of a disease or condition **characterised by** the presence of damaged and/or senescent cells.

14. A nanoparticle according to any one of claims 1 to 10, or a cell according to claim 11, for use in cosmetics.

15. A method of identifying damaged and/or senescent cells, the method comprising administering a nanoparticle according to any one of claims 1 to 10, or a cell according to claim 11, and detecting the presence of damaged and/or senescent cells by detecting the presence of the payload.
